(19) Europäisches Patentamt
European Patent Office
Office européen des brevets

(11) EP 2 885 003 B1

(12) **EUROPEAN PATENT SPECIFICATION**

(45) Date of publication and mention of the grant of the patent:
**29.09.2021 Bulletin 2021/39**

(21) Application number: **13751058.2**

(22) Date of filing: **14.08.2013**

(51) Int Cl.:
***A61K 45/06*** *(2006.01)* ***A61K 31/427*** *(2006.01)*
***A61K 31/4745*** *(2006.01)* ***A61K 31/5377*** *(2006.01)*
***A61P 35/00*** *(2006.01)*

(86) International application number:
**PCT/US2013/054848**

(87) International publication number:
**WO 2014/028566 (20.02.2014 Gazette 2014/08)**

(54) **COMBINATION OF PI3K INHIBITOR AND C-MET INHIBITOR**

KOMBINATION VON PI3K INHIBITOR UND C-MET INHIBITOR

COMBINAISON DE PI3K INHIBITEUR ET DE C-MET INHIBITEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2012 US 201261683790 P**

(43) Date of publication of application:
**24.06.2015 Bulletin 2015/26**

(73) Proprietor: **Novartis AG**
**4056 Basel (CH)**

(72) Inventors:
- **CAPONIGRO, Giordano**
**Cambridge, Massachusetts 02139 (US)**
- **HUANG, Xizhong**
**Cambridge, Massachusetts 02139 (US)**
- **LEHAR, Joseph**
**Cambridge, Massachusetts 02139 (US)**
- **WANG, Hui-Qin**
**Cambridge, Massachusetts 02139 (US)**

(74) Representative: **Rudge, Sewkian**
**Novartis Pharma AG**
**Patent Department**
**4002 Basel (CH)**

(56) References cited:
**WO-A1-2009/109605** **WO-A1-2012/062694**
**US-A1- 2010 267 742**

- **HUA C. GONG ET AL: "Signatures of Drug Sensitivity in Nonsmall Cell Lung Cancer", INTERNATIONAL JOURNAL OF PROTEOMICS, vol. 2011, 1 January 2011 (2011-01-01), pages 1-13, XP055030274, ISSN: 2090-2166, DOI: 10.1155/2011/215496**
- **HERBST ROY S ET AL: "Enzastaurin, a protein kinase Cbeta- selective inhibitor, and its potential application as an anticancer agent in lung cancer.", CLINICAL CANCER RESEARCH : AN OFFICIAL JOURNAL OF THE AMERICAN ASSOCIATION FOR CANCER RESEARCH 1 AUG 2007, vol. 13, no. 15 Pt 2, 1 August 2007 (2007-08-01), pages s4641-s4646, XP002714760, ISSN: 1078-0432**
- **MUKHERJEE BIPASHA ET AL: "The dual PI3K/mTOR inhibitor NVP-BEZ235 is a potent inhibitor of ATM- and DNA-PKCs-mediated DNA damage responses.", NEOPLASIA (NEW YORK, N.Y.) JAN 2012, vol. 14, no. 1, January 2012 (2012-01), pages 34-43, XP002714761, ISSN: 1476-5586**
- **X. LIU ET AL: "A Novel Kinase Inhibitor, INCB28060, Blocks c-MET-Dependent Signaling, Neoplastic Activities, and Cross-Talk with EGFR and HER-3", CLINICAL CANCER RESEARCH, vol. 17, no. 22, 15 November 2011 (2011-11-15), pages 7127-7138, XP055030185, ISSN: 1078-0432, DOI: 10.1158/1078-0432.CCR-11-1157**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- CHRISTOPHER R. ZITO ET AL: "Multi-Level Targeting of the Phosphatidylinositol-3-Kinase Pathway in Non-Small Cell Lung Cancer Cells", PLOS ONE, vol. 7, no. 2, 15 February 2012 (2012-02-15), page e31331, XP55095269, DOI: 10.1371/journal.pone.0031331
- D. KOUL ET AL: "Antitumor Activity of NVP-BKM120--A Selective Pan Class I PI3 Kinase Inhibitor Showed Differential Forms of Cell Death Based on p53 Status of Glioma Cells", CLINICAL CANCER RESEARCH, vol. 18, no. 1, 7 November 2011 (2011-11-07), pages 184-195, XP55095266, ISSN: 1078-0432, DOI: 10.1158/1078-0432.CCR-11-1558
- BARTHOLOMEUSZ CHANDRA ET AL: "Targeting the PI3K signaling pathway in cancer therapy", EXPERT OPINION ON THERAPEUTIC TARGETS, ASHLEY PUBLICATIONS, LONDON, GB, vol. 16, no. 1, 1 January 2012 (2012-01-01), pages 121-130, XP008157845, ISSN: 1472-8222, DOI: 10.1517/14728222.2011.644788
- None

## Description

### Field of the Invention

The present invention relates to a pharmaceutical combination which comprises (a) a phosphatidylinositol 3-kinase inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or pharmaceutically acceptable salt thereof, and (b) at least one c-Met receptor tyrosine kinase inhibitor, or pharmaceutically acceptable salt thereof, for use in the treatment of a proliferative disease having a dysregulation of c-Met and/or the HGF/c-Met signaling pathway by HGF / c-Met gene overexpression or amplification, HGF-dependent autocrine and paracrine activation and/or c-Met receptor activation through a genetic mutation. The present invention also relates to a pharmaceutical composition comprising such a pharmaceutial combination and a pharmaceutically acceptable carrier; a pharmaceutical combination comprising COMPOUND D, or a pharmaceutically acceptable salt thereof, and 2-fluoro-N-methyl-4-[7-quinolin-6-yl-methyl)-imidazo[1,2-b][1,2,4]triazin-2-yl]benzamide, or pharmaceutically acceptable salt thereof; and COMPOUND D for use in the treatment of a proliferative disease having a dysregulation of c-Met and/or the HGF/c-Met signalling pathway by HGF / c-Met gene overexpression or amplification, HGF-dependent autocrine and paracrine activation and/or c-Met receptor activation through a genetic mutation in combination with at least one c-Met receptor tyrosine kinase inhibitor, or a pharmaceutically acceptable salt thereof. It also relates to a c-Met receptor tyrosine kinase inhibitor which is 2-fluoro-N-methyl-4-[7-quinolin-6-yl-methyl)-imidazo[1,2-b][1,2,4]triazin-2yl]benzamide, or a pharmaceutically acceptable salt thereof, for use in the treatment of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with COMPOUND D, or a pharmaceutically acceptable salt thereof.

### Background

Protein kinases (PKs) are a group of enzymes that regulate diverse, important biological processes including cell growth, survival and differentiation, organ formation and morphogenesis, neovascularization, tissue repair and regeneration, among others. Protein kinases exert their physiological functions through catalyzing the phosphorylation of proteins (or substrates) and thereby modulating the cellular activities of the substrates in various biological contexts.

Protein kinases can be categorized as receptor type and non-receptor type. Receptor tyrosine kinases (RTKs) have an extracellular portion, a transmembrane domain, and an intracellular portion, while non-receptor tyrosine kinases are entirely intracellular. RTK mediated signal transduction is typically initiated by extracellular interaction with a specific growth factor (ligand), typically followed by receptor dimerization, stimulation of the intrinsic protein tyrosine kinase activity, and receptor transphosphorylation. Binding sites are thereby created for intracellular signal transduction molecules and lead to the formation of complexes with a spectrum of cytoplasmic signaling molecules that facilitate the appropriate cellular response such as cell division, differentiation, metabolic effects, and changes in the extracellular microenvironment. The non-receptor type of tyrosine kinases is also composed of numerous subfamilies, including Src, Btk, Abl, Fak, and Jak.

c-Met, a proto-oncogene, is a member of a distinct subfamily of heterodimeric receptor tyrosine kinases which include Met, Ron, and Sea (Birchmeier, C. et al., Nat. Rev. Mol. Cell Biol. 2003, 4(12):915-925; Christensen, J. G. et al., Cancer Lett. 2005, 225(1):1-26). The only high affinity ligand for c-Met is the hepatocyte growth factor (HGF), also known as scatter factor (SF). Binding of HGF to c-Met induces activation of the receptor via autophosphorylation resulting in an increase of receptor dependent signaling. Both c-Met and HGF are widely expressed in a variety of organs, but their expression is normally confined to the cells of epithelial and mesenchymal origin, respectively. The biological functions of c-Met (or c-Met signaling pathway) in normal tissues and human malignancies such as cancer have been well documented (Christensen, J.G. et al., Cancer Lett. 2005, 225(1):1-26; Corso, S. et al., Trends in Mol. Med. 2005, 11(6):284-292).

HGF and c-Met are each required for normal mammalian development, and abnormalities reported in both HGF- and c-Met-null mice are consistent with proximity of embryonic expression and epithelial-mesenchymal transition defects during organ morphogenesis (Christensen, J.G. et al., Cancer Lett. 2005, 225(1):1-26). Consistent with these findings, the transduction of signaling and subsequent biological effects of HGF/c-Met pathway have been shown to be important for epithelial-mesenchymal interaction and regulation of cell migration, invasion, cell proliferation and survival, angiogenesis, morphogenesis and organization of three-dimensional tubular structures (e.g. renal tubular cells, gland formation) during development. The specific consequences of c-Met pathway activation in a given cell/tissue are highly context-dependent.

Evidence shows that dysregulated c-Met pathway plays important and sometimes causative (in the case of genetic alterations) roles in tumor formation, growth, maintenance and progression (Birchmeier, C. et al., Nat. Rev. Mol. Cell. Biol. 2003, 4(12):915-925; Boccaccio, C. et al., Nat. Rev. Cancer 2006, 6(8):637-645; Christensen, J.G. et al.,

Cancer Lett. 2005, 225(1):1-26). HGF and/or c-Met are overexpressed in significant portions of most human cancers, and are often associated with poor clinical outcomes such as more aggressive disease, disease progression, tumor metastasis and shortened patient survival. Further, patients with high levels of HGF/c-Met proteins are more resistance to chemotherapy and radiotherapy. In addition to the abnormal HGF/c-Met expression, c-Met receptor can also be activated in cancer patients through genetic mutations (both germline and somatic) and gene amplification. Although gene amplification and mutations are the most common genetic alterations that have been reported in patients, the receptor can also be activated by deletions, truncations, gene rearrangement, as well as abnormal receptor processing and defective negative regulatory mechanisms.

Further, upon activation, c-MET activates a diverse number of intracellular signaling pathways, including but not limited to the phosphatidylinositol 3-kinase (PI3K) / Akt pathway, ERK 1 / 2, p38, and STAT3. PI3Ks comprise a family of lipid and serine/threonine kinases that catalyze the transfer of phosphate to the D-3' position of inositol lipids to produce phosphoinositol-3-phosphate (PIP), phosphoinositol-3,4-diphosphate (PIP2) and phosphoinositol-3,4,5-triphosphate (PIP3) that, in turn, act as second messengers in signaling cascades by docking proteins containing pleckstrin-homology, FYVE, Phox and other phospholipid-binding domains into a variety of signaling complexes often at the plasma membrane (Vanhaesebroeck et al., Annu. Rev. Biochem 70:535 (2001); Katso et al., Annu. Rev. Cell Dev. Biol. 17:615 (2001)). Of the two Class 1 PI3Ks, Class 1A PI3Ks are heterodimers composed of a catalytic p110 subunit (α, β, δ isoforms) constitutively associated with a regulatory subunit that can be p85α, p55α, p50α, p85β or p55γ. The Class 1B sub-class has one family member, a heterodimer composed of a catalytic p110y subunit associated with one of two regulatory subunits, p101 or p84 (Fruman et al., Annu Rev. Biochem. 67:481 (1998); Suire et al., Curr. Biol. 15:566 (2005)). The modular domains of the p85/55/50 subunits include Src Homology (SH2) domains that bind phosphotyrosine residues in a specific sequence context on activated receptor and cytoplasmic tyrosine kinases, resulting in activation and localization of Class 1A PI3Ks. Class 1B PI3K is activated directly by G protein-coupled receptors that bind a diverse repertoire of peptide and non-peptide ligands (Stephens et al., Cell 89:105 (1997)); Katso et al., Annu. Rev. Cell Dev. Biol. 17:615-675 (2001)). The PI3K pathway is a crucial regulator of key cellular processes including proliferation, survival, chemotaxis, cellular trafficking, motility, metabolism, inflammatory and allergic responses, transcription and translation (Cantley et al., Cell 64:281 (1991); Escobedo and Williams, Nature 335: 85 (1988); Fantl et al., Cell., 69: 413 (1992). 2-carboxamide cycloamino urea derivatives such as (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) (COMPOUND D) were shown to have effect in BT474 and HBCx-5 breast cancer and NCI-N87 gastric xenograft models (WO2012/062694). Bartholomeusz et al. reported in Expert Opin. Ther. Targets 2012, 16(1), 121 that the COMPOUND D is in Phase I clinical trials involving patients with advanced solid tumors with a mutation of the PIK3CA gene. Gong et al. disclosed in International journal of proteomics 2011, 2011, 1, that growth inhibition of H1993 lung tumor cells can be increased by combining BEZ-235 and PF-2341066. US2010/267742 disclosed a pharmaceutical composition including a PKCβ kinase inhibitor and c-met kinase inhibitor as useful in treating cancer, for example lung cancer.

In spite of significant advances in medicine and numerous treatment options for patients with cancer, there remains a need for effective and safe therapeutic agents and a need for new combination therapies that can be administered for the effective long-term treatment of cancer.

It is now found that the pharmaceutical combination comprising (a) a phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or pharmaceutically acceptable salt thereof, and (b) at least one c-Met receptor tyrosine kinase inhibitor, particularly 2-fluoro-N-methyl-4-[7-quinolin-6-yl-methyl)-imidazo[1,2-b][1,2,4]triazin-2yl]benzamide, or a pharmaceutically acceptable salt thereof is particularly effective for the treatment of proliferative disease having a dysregulation of c-Met and/or the HGF/c-Met signaling pathway.

It is expected that the anti-proliferative effect of this combination is greater than the maximum effect that can be achieved with either type of ingredient alone.

## Summary of the Invention

The present invention pertains in a first aspect to a pharmaceutical combination comprising (a) a phosphatidylinositol 3-kinase (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or pharmaceutically acceptable salt thereof, and (b) at least one c-Met receptor tyrosine kinase inhibitor or pharmaceutically acceptable salt thereof, for use in the treatment (via simultaneous, separate or sequential administration) of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation. The combination of the invention is particularly useful for the treatment of lung cancer (e.g., non-small cell lung cancer) or glioblastoma.

In a preferred embodiment, the c-Met receptor tyrosine kinase inhibitor is 2-fluoro-N-methyl-4-[7-quinolin-6-yl-methyl)-imidazo[1,2-b][1,2,4]triazin-2yl]benzamide or pharmaceutically acceptable salt thereof.

In another aspect, the present invention relates to (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) for use in the treatment of proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with at least one c-Met receptor tyrosine kinase inhibitor, or a pharmaceutically acceptable salt thereof.

In another aspect, the present invention relates to a c-Met receptor tyrosine kinase inhibitor which is 2-fluoro-N-methyl-4-[7-quinolin-6-yl-methyl)-imidazo[1,2-b][1,2,4]triazin-2yl]benzamide, or a pharmaceutically acceptable salt thereof, for use in the treatment of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or a pharmaceutically acceptable salt thereof.

The treatment of a profilerative disease can comprise inhibiting the formation of metastases.In a preferred embodiment, the proliferative disease is cancer.

In one aspect, the invention provides a pharmaceutical composition comprising a quantity, which is jointly therapeutically effective against a c-Met dependent proliferative disease, of the combination of COMPOUND D and at least one c-Met receptor tyrosine kinase inhibitor or a pharmaceutically acceptable salt thereof and one or more pharmaceutically acceptable carriers. This pharmaceutical composition can be administered to a subject as a fixed combination in a single formulation or unit dosage form by any suitable route. Alternatively, the therapeutic agents can be administered to a subject as a non-fixed combination in separate pharmaceutical compositions or formulations or unit dosage forms and administered either simultaneously, concurrently or sequentially with no specific time limits.

In one aspect, the present invention provides a commercial package comprising as therapeutic agents the pharmaceutical combination of Compoud D and at least one c-Met gene overexpression or amplification, HGF-dependent autocrine or paracrine activation and/or c-Met receptor activation through a genetic mutation, together with instructions for the simultaneous, separate or sequential administration thereof in the treatment of a proliferative disease having a dysregulation of c-Met and/or the HGF/c-Met signalling pathway by HGF/c-Met gene overexpression or amplification, HGF-dependent autocrine and paracrine activation and/or c-Met receptor activation through a genetic mutatuion. In one embodiment, the proliferative disease is lung cancer (e.g., non-small cell lung cancer) or glioblastoma.

In another aspect, the present invention provides a pharmaceutical composition comprising the combination of COMPOUND D and at least one c-Met receptor tyrosine kinase inhibitor or a pharmaceutically acceptable salt thereof and at least one pharmaceutically acceptable carrier.

Yet in another aspect, the present invention provides a pharmaceutical combination comprising (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or a pharmaceutically acceptable salt thereof and a c-Met receptor tyrosine kinase inhibitor 2-fluoro-N-methyl-4-[7-quinolin-6-yl-methyl)-imidazo[1,2-b][1,2,4]triazin-2yl]benzamide or a pharmaceutically acceptable salt thereof.

Yet in another aspect, the present invention provides compound D for use in the treatment of proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with at least one c-Met receptor tyrosine kinase inhibitor, or a pharmaceutically acceptable salt thereof.

Detailed Description of the Figures

Figure 1 shows effects of combining COMPOUND C and 2-fluoro-N-methyl-4-[7-quinolin-6-yl-methyl)-imidazo[1,2-b][1,2,4]triazin-2yl]benzamide doses on proliferation of H4 human glioblastoma tumor models.

Figure 2 shows effects of combining COMPOUND C and 2-fluoro-N-methyl-4-[7-quinolin-6-yl-methyl)-imidazo[1,2-b][1,2,4]triazin-2yl]benzamide doses on proliferation of U87-MG human glioblastoma tumor models.

Figure 3 shows effects of combining COMPOUND C and 2-fluoro-N-methyl-4-[7-quinolin-6-yl-methyl)-imidazo[1,2-b][1,2,4]triazin-2yl]benzamide doses on proliferation of A172 human glioblastoma tumor models.

Figure 4 shows effects of combining COMPOUND C and 2-fluoro-N-methyl-4-[7-quinolin-6-yl-methyl)-imidazo[1,2-b][1,2,4]triazin-2yl]benzamide doses on proliferation of LN229 human glioblastoma tumor models.

Figure 5 shows the comparative mean anti-tumor growth effects for the combination 2-fluoro-N-methyl-4-[7-quinolin-6-yl-methyl)-imidazo[1,2-b][1,2,4]triazin-2yl]benzamide and COMPOUND D in NCI-H1993 human non-small cell lung cancer xenograft models as compared to vehicle control and both monotherapies.

Detailed Description

The present invention pertains to a pharmaceutical combination comprising (a) a phosphatidylinositol 3-kinase (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or a pharmaceutically acceptable salt thereof, and (b) at least one c-Met receptor tyrosine kinase inhibitor or pharmaceutically acceptable salt thereof, for for use (via simultaneous, separate or sequential administration) in the treatment of a proliferative disease having a dysregulation of c-Met and/or the HGF/c-Met signalling pathway by HGF/c-Met gene overexpression or amplification, HGF-dependent autocrine and paracrine activation, and/or c-Met receptor activation through a genetic mutation. The combination of the invention is particularly useful for the treatment of lung cancer (e.g., non-small cell lung cancer) or glioblastoma.

The general terms used herein are defined with the following meanings, unless explicitly stated otherwise:

The terms "comprising" and "including" are used herein in their open-ended and nonlimiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt.

The term "combination" or "pharmaceutical combination" is defined herein to refer to either a fixed combination in one dosage unit form, a non-fixed combination or a kit of parts for the combined administration where the phosphatidylinositol 3-kinase inhibitor (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) (COMPOUND D) and the c-Met receptor tyrosine kinase inhibitor or pharmaceutically acceptable salt thereof may be administered independently at the same time or separately within time intervals that allow that the therapeutic agents show a cooperative, e.g., synergistic, effect.

The term "fixed combination" means that the therapeutic agents, e.g. the phosphatidylinositol 3-kinase inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) and the c-Met receptor tyrosine kinase inhibitor, are administered to a subject simultaneously in the form of a single entity or dosage form.

The term "non-fixed combination" means that the therapeutic agents, e.g. the phosphatidylinositol 3-kinase inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) and the c-Met receptor tyrosine kinase inhibitor or pharmaceutically acceptable salt thereof, are both administered to a patient as separate entities or dosage forms either simultaneously, concurrently or sequentially with no specific time limits, wherein such administration provides therapeutically effective levels of the compounds in the body of the subject, e.g., a mammal or human, in need thereof.

The term "kit of parts" refers to the therapeutic agents (a) and (b) as defined above that are dosed independently or by use of different fixed combinations with distinguished amounts of the therapeutic agents (a) and (b), i.e., simultaneously or at different time points. The parts of the kit of parts can then e.g., be administered simultaneously or chronologically staggered, that is at different time points and with equal or different time intervals for any part of the kit of parts. The ratio of the total amounts of the therapeutic agent (a) to the therapeutic agent (b) to be administered in the combined preparation can be varied, e.g., in order to cope with the needs of a patient sub-population to be treated or the needs of the single patient.

The term "a phosphatidylinositol 3-kinase inhibitor" or "PI3K inhibitor" is defined herein to refer to a compound which targets, decreases or inhibits phosphatidylinositol 3-kinase. Phosphatidylinositol 3-kinase activity has been shown to increase in response to a number of hormonal and growth factor stimuli, including insulin, platelet-derived growth factor, insulin-like growth factor, epidermal growth factor, colony-stimulating factor, and hepatocyte growth factor, and has been implicated in processes related to cellular growth and transformation.

The term "c-Met receptor tyrosine kinase inhibitor" is defined herein to refer to a compound which targets, decreases, or inhibitor activity of the c-Met receptor tyrosine kinase.

The term "c-Met dependent proliferative disease" or "c-Met dependent cancer" refers to those proliferative diseases having a dysregulation of c-Met and/or the HGF/c-Met signaling pathway, particularly HGF/ c-Met gene overexpression and amplification, HGF-dependent autocrine and paracrine activation, and/or c-Met receptor genetic mutations (both germline and somatic). In a preferred embodiment, the proliferative disease is a cancer having a dysregulation of the c-Met and/or the HGF/ c-Met signaling pathway. The "dysregulation of c-Met and/or the HGF/c-Met signaling pathway" is meant to include activation of the c-Met class of receptor tyrosine kinases through various mechanisms including, but not limited to, HGF-dependent autocrine and paracrine activation, HGF/ c-met gene overexpression and/or amplification, point mutations, deletions, truncations, rearrangement, as well as abnormal c-Met receptor processing and defective negative regulatory mechanisms. In a preferred embodiment, the term "c-Met dependent proliferative disease" or "c-Met dependent cancer" includes HGF/ c-Met gene overexpression and amplification.

The term "pharmaceutical composition" is defined herein to refer to a mixture or solution containing at least one therapeutic agent to be administered to a subject, e.g., a mammal or human, in order to treat a particular disease or condition affecting the subject thereof.

The term "pharmaceutically acceptable" is defined herein to refer to those compounds, materials, compositions and/or dosage forms, which are, within the scope of sound medical judgment, suitable for contact with the tissues of a subject, e.g., a mammal or human, without excessive toxicity, irritation allergic response and other problem complications commensurate with a reasonable benefit / risk ratio.

The term "treating" or "treatment" as used herein comprises a treatment relieving, reducing or alleviating at least one symptom in a subject or effecting a delay of progression of a disease. For example, treatment can be the diminishment of one or several symptoms of a disorder or complete eradication of a disorder, such as cancer. Within the meaning of the present invention, the term "treat" also denotes to arrest, delay the onset (i.e., the period prior to clinical manifestation of a disease) and/or reduce the risk of developing or worsening a disease.

The term "jointly therapeutically active" or "joint therapeutic effect" as used herein means that the therapeutic agents may be given separately (in a chronologically staggered manner, especially a sequence-specific manner) in such time intervals that they prefer, in the warm-blooded animal, especially human, to be treated, still show a (preferably synergistic) interaction (joint therapeutic effect). Whether this is the case can, *inter alia,* be determined by following the blood levels, showing that both therapeutic agents are present in the blood of the human to be treated at least during certain time intervals.

The term "pharmaceutically effective amount" or "clinically effective amount" of a pharmaceutical combination of therapeutic agents is an amount sufficient to provide an observable improvement over the baseline clinically observable signs and symptoms of the proliferative disease treated with the combination.

The term "synergistic effect" as used herein refers to action of two therapeutic agents such as, for example, COMPOUND D or a pharmaceutically acceptable salt thereof and c-Met receptor tyrosine kinase inhibitor or pharmaceutically acceptable salt thereof producing an effect, for example, slowing the symptomatic progression of a proliferative disease, particularly cancer, or symptoms thereof, which is greater than the simple addition of the effects of each therapeutic agent administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

The term "subject" or "patient" as used herein includes animals, which are capable of suffering from or afflicted with a proliferative disease or any disorder involving, directly or indirectly, a tumor. Examples of subjects include mammals, e.g., humans, dogs, cows, horses, pigs, sheep, goats, cats, mice, rabbits, rats and transgenic non-human animals. In the preferred embodiment, the subject is a human, e.g., a human suffering from, at risk of suffering from, or potentially capable of suffering from a proliferative disease.

The term about" or "approximately" shall have the meaning of within 10%, more preferably within 5%, of a given value or range.

Combinations of the present invention include a PI3K inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or pharmaceutically acceptable salts thereof.

WO2010/029082 describes specific 2-carboxamide cycloamino urea derivatives, which have been found to have inhibitory activity for the α -isoform of PI3K. Specific 2-carboxamide cycloamino urea derivative (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) which is suitable for the combination of the present invention, its preparation and suitable formulations containing the same are described in WO2010/029082.

The synthesis of COMPOUND D is described in WO2010/029082 as Example 15.

COMPOUND D may be used in form of the free base or a pharmaceutically acceptable salt thereof. Suitable salts include those formed, for example, as acid addition salts, preferably with organic or inorganic acids, with a basic nitrogen atom. Suitable inorganic acids are, for example, halogen acids, such as hydrochloric acid, sulfuric acid, or phosphoric acid. Suitable organic acids are, e.g., carboxylic acids or sulfonic acids, such as fumaric acid or methansulfonic acid.

Combinations of the present invention further include at least one c-Met receptor tyrosine kinase inhibitor or pharmaceutically acceptable salt thereof. Examples of suitable c-Met receptor tyrosine kinase inhibitor include, but are not limited to, 2-fluoro-N-methyl-4-[7-quinolin-6-yl-methyl)-imidazo[1,2-b][1,2,4]triazin-2yl]benzamide, ARQ197 (tavantinib), AMG458, GSK1363089 (XL880 or foretinib), PF2341066 (crizotinib), or a pharmaceutically acceptable salt thereof.

Preferably, the c-Met receptor tyrosine kinase inhibitor for use in the combination of the present invention is a compound of formula (IV)

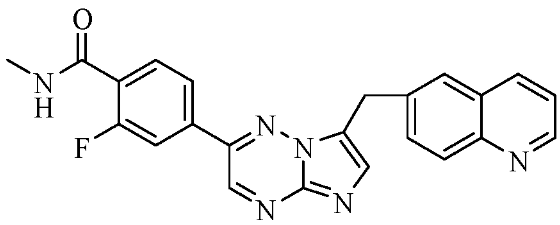

(IV),

or a pharmaceutically acceptable salt thereof. The compound of formula (IV) has a chemical name of 2-fluoro-N-methyl-4-[7-quinolin-6-yl-methyl)-imidazo[1,2-b][1,2,4]triazin-2yl]benzamide. This compound and the method for synthesis of this compound is disclosed in Example 7 of International PCT patent application WO2008/064157.

The compound of formula (IV) may be used in form of the free base or a pharmaceutically acceptable salt thereof. Suitable salts of 2-fluoro-N-methyl-4-[7-quinolin-6-yl-methyl)-imidazo[1,2-b][1,2,4]triazin-2yl]benzamide include mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. Preferably, the compound of formula (IV) is a salt form disclosed in PCT patent application WO2009/143211. Preferred salt forms of the compound of formula (IV) include the dihydrochloric acid salt form and the dibenzensulfonic acid salt form of 2-fluoro-N-methyl-4-[7-quinolin-6-yl-methyl)-imidazo[1,2-b][1,2,4]triazin-2yl]benzamide.

Suitable c-Met receptor tyrosine kinase inhibitors further include:

(i.) ARQ197 (tavantinib)(developed by Daiichi Sankyo and ArQule) having chemical structure:

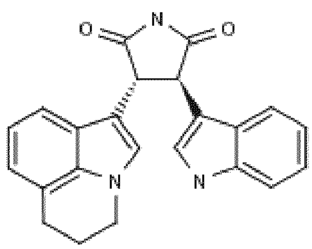

;

(ii.) AMG458 (developed by Amgen) having chemical structure:

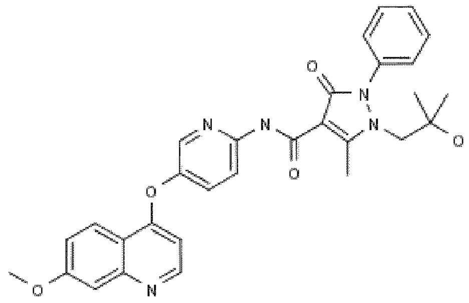

(iii.) GSK1363089 (also known as XL880 or foretinib) (developed by GlaxoSmithKline) having chemical structure:

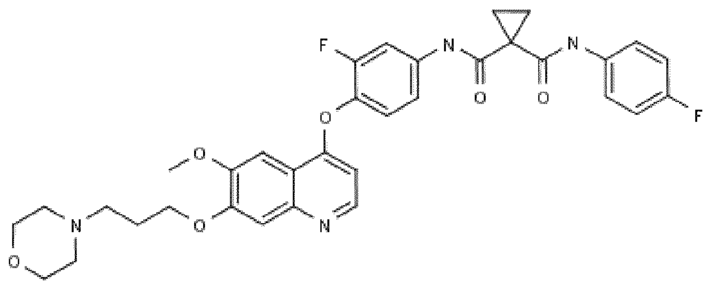

;

and

(iv.) PF2341066 (also known as crizotinib) (developed by Pfizer) having chemical structure:

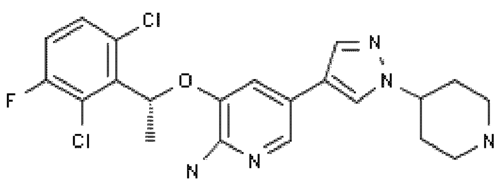

The structure of the therapeutic agents identified by code nos., generic or trade names may be taken from the actual edition of the standard compendium "The Merck Index" or from databases, e.g., Patents International (e.g, IMS World Publications).

A pharmaceutical combination comprising (a) a phosphatidylinositol 3-kinase (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) (COMPOUND D) or pharmaceutically acceptable salt thereof, and (b) at least one c-Met receptor tyrosine kinase inhibitor or pharmaceutically acceptable salt thereof, will be referred to hereinafter as a COMBINATION OF THE INVENTION.

Unless otherwise specified, or clearly indicated by the text, or not applicable, reference to therapeutic agents useful in the COMBINATION OF THE INVENTION includes both the free base of the compounds, and all pharmaceutically acceptable salts of the compounds.

In one preferred embodiment of the present invention, the combination comprises (a) a phosphatidylinositol 3-kinase inhibitor COMPOUND D or a pharmaceutically acceptable salt thereof, and (b) 2-fluoro-N-methyl-4-[7-quinolin-6-yl-methyl)-imidazo[1,2-b][1,2,4]triazin-2yl]benzamide or a pharmaceutically acceptable salt thereof.

The COMBINATION OF THE INVENTION is useful for the treatment of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation in a subject in need thereof. The COMBINATION OF THE INVENTION may be used for the treatment of a proliferative disease in a subject in need thereof by administering to the subject a pharmaceutical combination comprising (a) an effective amount of (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or pharmaceutically acceptable salt thereof, and (b) an effective amount of at least one c-Met receptor tyrosine kinase inhibitor, such as 2-fluoro-N-methyl-4-[7-quinolin-6-yl-methyl)-imidazo[1,2-b][1,2,4]triazin-2yl]benzamide, or pharmaceutically acceptable salt thereof. Preferably, these therapeutic agents are administered at therapeutically effective dosages which, when combined, provide a jointly beneficial effect. The administration may be separate, simultaneous or sequential.

The nature of proliferative diseases, particularly c-Met dependent proliferative diseases, is multifactorial. Under certain circumstances, drugs with different mechanisms of action may be combined. However, just considering any combination of drugs having different mode of action does not necessarily lead to combinations with advantageous effects.

It has been found that the administration of the COMBINATION OF THE INVENTION may be used to treat a subject having a proliferative disease, particularly lung cancer (e.g., non-small cell lung cancer) or glioblastoma. In the present invention, the administration of the COMBINATION OF THE INVENTION results in a more beneficial treatment, e.g, synergistic or improved anti-proliferative effect, e.g., with regard to the delay of progression of a proliferative disease or with regard to a change in tumor volume, as compared to either monotherapy.

In one embodiment, the proliferative disease having dysregulation of a c-Met and/or HGF/c-Met signalling pathway is a cancer having dysregulation of c-Met and/or the HGF/c-Met signaling pathway. In a further embodiment, this proliferative disease is a cancer having HGF/ c-Met gene overexpression and/or amplification.

C-Met dependent proliferative diseases, suitable for treatment with the COMBINATION OF THE INVENTION include, but are not limited to cancers, atherosclerosis, lung fibrosis, renal fibrosis and regeneration, liver diseases, allergic disorders, inflammatory and autoimmune disorders, cerebrovascular diseases, cardiovascular diseases, and conditions associated with organ transplantation.

In one embodiment, the c-Met dependent proliferative disease is cancer. The term "cancer" is used herein to mean a broad spectrum of tumors, including all solid and hematological malignancies. Examples of such tumors include but are not limited to benign and malignant tumors of the breast, bladder, cervix, cholangiocarcinoma, colorectum, esophagus, gastric, head and neck, kidney (e.g., papillary renal cell carcinoma), liver (e.g, hepatocellular carcinoma), lung (e.g., small-cell lung cancer and non-small cell lung cancer), nasopharygeal, ovary, pancreas, prostate, thyroid, endometrial, sarcomas of the musculoskeletal system (e.g., osteosarcaoma, synovial sarcoma, rhabdomyosarcoma), sarcomas of soft tissues sarcomas (e.g., MFH/fibrosarcoma, leiomyosarcoma, Kaposi’s sarcoma), multiple myeloma, lymphomas, adult T cell leukemia, acute myelogenous leukemia, chronic myeloid leukemia, glioblastomas, astrocytomas, melanoma, mesothelioma and Wilm’s tumor and the like.

In a further embodiment of the present invention, the c-Met dependent proliferative disease is a solid tumor. The term "solid tumor" especially means breast cancer, bladder cancer, ovarian cancer, colorectal cancer, melanoma, gastric cancer, cervical cancer, lung cancer (e.g., small-cell lung cancer and non-small cell lung cancer), head and neck cancer, prostate cancer, or Kaposi’s sarcoma. The present combination inhibits the growth of solid tumors and also liquid tumors.

In a further embodiment of the present invention, the c-Met dependent proliferative disease is lung cancer (e.g., non-small cell lung cancer), or glioblastoma.

In a further embodiment, the present invention pertains to the COMBINATION OF THE INVENTION for use in the treatment of a p c-Met dependent proliferative disease, that is resistant to treatment with a c-Met receptor tyrosine kinase inhibitor, particularly 2-fluoro-N-methyl-4-[7-quinolin-6-yl-methyl)-imidazo[1,2-b][1,2,4]triazin-2yl]benzamide, or a pharmaceutically acceptable salt thereof. As used herein, the term "resistant" means formerly sensitive tumors which, in the continuous presence of a c-Met receptor tyrosine kinase inhibitor, either have regrown after shrinking due to treatment, or have reappeared after being temporarily eliminated due to treatment. Successful treatment of resistant tumors can engender, e.g., increased sensitivity of a tumor cell to novel or previously attempted anti-cancer therapy and/or chemotherapeutic agents, and can result in, e.g., subsequent tumor cell death and prevention from metastasis.

In a further embodiment, the present invention pertains to a COMBINATION OF THE INVENTION for use in inhibiting the growth and/or spread of metastases of c-Met dependent cancers, particularly c-Met dependent tumors. The COMBINATION OF THE INVENTION is suitable for treatment of poor prognosis patients, especially such poor prognosis patients having metastatic lung cancer (e.g., non-small cell lung cancer) or glioblastoma.

In a further embodiment, the present invention pertains to a pharmaceutical combination comprising (a) a phosphatidylinositol 3-kinase inhibitor COMPOUND D or a pharmaceutically acceptable salt thereof, and (b) the c-Met receptor tyrosine kinase inhibitor 2-fluoro-N-methyl-4-[7-quinolin-6-yl-methyl)-imidazo[1,2-b][1,2,4]triazin-2yl]benzamide or a pharmaceutically acceptable salt thereof for use in the treatment of a proliferative disease, particularly a c-Met dependent proliferative disease. Preferably, the proliferative disease is cancer, most preferably lung cancer (e.g., non-small cell lung cancer), or glioblastoma.

A patient having a c-Met dependent proliferative disease, particularly lung cancer (e.g., non-small cell lung cancer), or glioblastoma, may be separately, simultaneously or sequentially administered a combination comprising (a) a phosphatidylinositol 3-kinase (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) (COMPOUND D) or pharmaceutically acceptable salt thereof, and (b) at least one c-Met receptor tyrosine kinase inhibitor or pharmaceutically acceptable salt thereof for the treatment of said c-Met dependent proliferative disease in accordance with the present invention.

In a further embodiment, the present invention discloses COMPOUND D for use in a method for treating a c-Met dependent proliferative disease, the method comprising administering to subject in need thereof a combination of

(a) a phosphatidylinositol 3-kinase inhibitor COMPOUND D or a pharmaceutically acceptable salt thereof, and (b) the c-Met receptor tyrosine kinase inhibitor 2-fluoro-N-methyl-4-[7-quinolin-6-yl-methyl)-imidazo[1,2-b][1,2,4]triazin-2yl]benzamide or a pharmaceutically acceptable salt thereof in a quantity which is jointly therapeutically effective against said proliferative disease. Preferably, the c-Met dependent proliferative disease is cancer, most preferably lung cancer (e.g., non-small cell lung cancer), or glioblastoma.

In a further embodiment, the present invention relates to a COMPOUND D for use in a method of inhibiting the formation of metastases in a subject having a c-Met dependent cancer comprising administering to a subject in need thereof a combination of (a) a phosphatidylinositol 3-kinase (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) or pharmaceutically acceptable salt thereof, and (b) at least one c-Met receptor tyrosine kinase inhibitor or pharmaceutically acceptable salt thereof in a quantity which is jointly therapeutically effective against said cancer. Suitable cancers are those set forth in the embodiments above..

In a preferred embodiment, the present methods and combinations can be used to treat lung cancer (e.g., non-small cell lung cancer), or glioblastoma.

In a further embodiment, the present invention relates to a phosphatidylinositol 3-kinase inhibitor COMPOUND D or a pharmaceutically acceptable salt thereof in combination with the c-MET receptor tyrosine kinase inhibitor 2-fluoro-N-methyl-4-[7-quinolin-6-yl-methyl)-imidazo[1,2-b][1,2,4]triazin-2yl]benzamide or pharmaceutically acceptable salt thereof for use in the treatment of a c-Met proliferative disease. In a preferred embodiment, the c-Met proliferative disease is cancer, particularly lung cancer (e.g., non-small cell lung cancer), or glioblastoma.

The administration of a COMBINATION OF THE INVENTION may result not only in a beneficial effect, e.g. therapeutic effect as compared to monotherapy of the individual therapeutic agents of the combination, *e.g,* a synergistic therapeutic effect, *e.g.* with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically therapeutic agents used in the combination of the invention.

A further benefit is that lower doses of the therapeutic agents of the COMBINATION OF THE INVENTION can be used, for example, that the dosages need not only often be smaller, but are also applied less frequently, or can be used in order to diminish the incidence of side-effects observed with one of the therapeutic agents alone. This is in accordance with the desires and requirements of the patients to be treated.

It can be shown by established test models that a COMBINATION OF THE INVENTION results in the beneficial effects described herein before. The person skilled in the art is fully enabled to select a relevant test model to prove such beneficial effects. The pharmacological activity of a COMBINATION OF THE INVENTION may, for example, be demonstrated in a clinical study or in an *in-vitro* test procedure as essentially described hereinafter.

Suitable clinical studies are in particular, for example, open label, dose escalation studies in patients with a c-Met proliferative disease, particularly lung cancer (e.g., non-small cell lung cancer) or glioblastoma. Such studies prove in particular the synergism of the therapeutic agents of the combination of the invention. The beneficial effects on c-Met dependent proliferative diseases may be determined directly through the results of these studies which are known as such to a person skilled in the art. Such studies may be, in particular, suitable to compare the effects of a monotherapy using either therapeutic agent and a pharmaceutical combination of the invention. In one embodiment, the dose of the phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) is escalated until the Maximum Tolerated Dosage is reached, and the c-Met receptor tyrosine kinase inhibitor, *e.g*, 2-fluoro-N-methyl-4-[7-quinolin-6-yl-methyl)-imidazo[1,2-b][1,2,4]triazin-2yl]benzamide, is administered with a fixed dose. Alternatively, phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) may be administered in a fixed dose and the dose of the c-MET receptor tyrosine kinase inhibitor, *e.g.*, 2-fluoro-N-methyl-4-[7-quinolin-6-yl-methyl)-imidazo[1,2-b][1,2,4]triazin-2yl]benzamide, may be escalated. Each patient may receive doses of the phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) either daily or intermittently. The efficacy of the treatment may be determined in such studies, *e.g.*, after 12, 18 or 24 weeks by evaluation of symptom scores every 6 weeks.

Determining a synergistic interaction between one or more components, the optimum range for the effect and absolute dose ranges of each component for the effect may be definitively measured by administration of the components over different w/w ratio ranges and doses to patients in need of treatment. For humans, the complexity and cost of carrying out clinical studies on patients may render impractical the use of this form of testing as a primary model for synergy. However, the observation of synergy in one species can be predictive of the effect in other species and animal models exist, as described herein, to measure a synergistic effect and the results of such studies can also be used to predict effective dose ratio ranges and the absolute doses and plasma concentrations required in other species by the application of pharmacokinetic/ pharmacodynamic methods. Established correlations between tumor models and effects seen in man suggest that synergy in animals may be demonstrated, for example, by xenograft models or in appropriate

cell lines.

Aspects of the present invention may employ combinations of phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) as formulated as pharmaceutical composition comprising one or more pharmaceutically acceptable carriers.

In one aspect, the invention provides a pharmaceutical composition comprising a quantity, which is jointly therapeutically effective against a c-Met dependent proliferative disease, of a COMBINATION OF THE INVENTION and one or more pharmaceutically acceptable carriers. This pharmaceutical composition may consist of the therapeutic agents (a) and (b) to be administered to a patient as a fixed combination in a single formulation or unit dosage form by any suitable route. Alternatively, the therapeutic agents, e.g. the phosphatidylinositol 3-kinase inhibitor (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D) and the c-Met receptor tyrosine kinase inhibitor or pharmaceutically acceptable salt thereof, are both to be administered to a patient as a non-fixed combination in separate pharmaceutical compositions or formulations or unit dosage forms and administered either simultaneously, concurrently or sequentially with no specific time limits.

In a preferred embodiment, the invention provides pharmaceutical compositions separately comprising a quantity, which is jointly therapeutically effective against a c-Met dependent proliferative disease, of therapeutic agent (a) and therapeutic agent (b) which are administered concurrently but separately, or administered sequentially to a subject in need thereof.

The pharmaceutical compositions for separate administration of the therapeutic agents or for the administration of the therapeutic agents in a fixed combination, *i.e.* a single galenical composition comprising the COMBINATION OF THE INVENTION, may be prepared in a manner known *per se* and are those suitable for enteral, such as oral or rectal, and parenteral administration to subjects (warm-blooded animals), including humans, comprising a therapeutically effective amount of at least one pharmacologically active therapeutic agent alone, *e.g.* as indicated above, or in combination with one or more pharmaceutically acceptable carriers, especially suitable for enteral or parenteral application.

The novel pharmaceutical composition may contain, from about 0.1 % to about 99.9%, preferably from about 1 % to about 60 %, of the therapeutic agent(s).

Pharmaceutical compositions for the combination therapy, including fixed combinations or non-fixed combinations, for enteral or parenteral administration are, for example, those in unit dosage forms, such as sugar-coated tablets, tablets, capsules or suppositories, or ampoules. If not indicated otherwise, these are prepared in a manner known *per se,* for example by means of various conventional mixing, comminution, granulating, sugar-coating, dissolving, lyophilizing processes, or fabrication techniques readily apparent to those skilled in the art. It will be appreciated that the unit content of a therapeutic agent contained in an individual dose of each dosage form need not in itself constitute an effective amount since the necessary effective amount may be reached by administration of a plurality of dosage units.

A unit dosage form containing the combination of agents or individual agents of the combination of agents may be in the form of micro-tablets enclosed inside a capsule, *e.g.* a gelatin capsule. For this, a gelatin capsule as is employed in pharmaceutical formulations can be used, such as the hard gelatin capsule known as CAPSUGEL, available from Pfizer.

The unit dosage forms of the present invention may optionally further comprise additional conventional carriers or excipients used for pharmaceuticals. Examples of such carriers include, but are not limited to, disintegrants, binders, lubricants, glidants, stabilizers, and fillers, diluents, colorants, flavours and preservatives. One of ordinary skill in the art may select one or more of the aforementioned carriers with respect to the particular desired properties of the dosage form by routine experimentation and without any undue burden. The amount of each carriers used may vary within ranges conventional in the art. The following references disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003).

These optional additional conventional carriers may be incorporated into the oral dosage form either by incorporating the one or more conventional carriers into the initial mixture before or during melt granulation or by combining the one or more conventional carriers with the granules in the oral dosage form. In the latter embodiment, the combined mixture may be further blended, e.g., through a V-blender, and subsequently compressed or molded into a tablet, for example a monolithic tablet, encapsulated by a capsule, or filled into a sachet.

Examples of pharmaceutically acceptable disintegrants include, but are not limited to, starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. The disintegrant may be present in an amount from about 0% to about 10% by weight of the composition. In one embodiment, the disintegrant is present in an amount from about 0.1% to about 5% by weight of composition.

Examples of pharmaceutically acceptable binders include, but are not limited to, starches; celluloses and derivatives thereof, for example, microcrystalline cellulose, e.g., AVICEL PH from FMC (Philadelphia, PA), hydroxypropyl

cellulose hydroxylethyl cellulose and hydroxylpropylmethyl cellulose METHOCEL from Dow Chemical Corp. (Midland, MI); sucrose; dextrose; corn syrup; polysaccharides; and gelatin. The binder may be present in an amount from about 0% to about 50%, e.g., 2-20% by weight of the composition.

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant may be present in an amount from about 0% to about 10% by weight of the composition. In one embodiment, the lubricant may be present in an amount from about 0.1% to about 1.5% by weight of composition. The glidant may be present in an amount from about 0.1% to about 10% by weight.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 0% to about 80% by weight of the composition.

In one embodiment, the pharmaceutical composition comprises a quantity which is jointly therapeutically effective against a c-Met dependent proliferative disease, of a COMBINATION OF THE INVENTION and one or more pharmaceutically acceptable carriers, wherein the phosphatidylinositol 3-kinase inhibitor is COMPOUND D or a pharmaceutically acceptable salt thereof.

In a further embodiment, the pharmaceutical composition comprises a quantity which is jointly therapeutically effective against a proliferative disease of COMBINATION OF THE INVENTION and one or more pharmaceutically acceptable carriers, wherein the c-Met inhibitor is 2-fluoro-N-methyl-4-[7-quinolin-6-yl-methyl)-imidazo[1,2-b][1,2,4]triazin-2yl]benzamide or a pharmaceutically acceptable salt thereof.

In a further embodiment, the pharmaceutical composition comprises a quantity which is jointly therapeutically effective against a proliferative disease of COMBINATION OF THE INVENTION and one or more pharmaceutically acceptable carriers, wherein the phosphatidylinositol 3-kinase inhibitor is COMPOUND D or a pharmaceutically acceptable salt thereof and the c-Met inhibitor is 2-fluoro-N-methyl-4-[7-quinolin-6-yl-methyl)-imidazo[1,2-b][1,2,4]triazin-2yl]benzamide or a pharmaceutically acceptable salt thereof.

In accordance with the present invention, a therapeutically effective amount of each of the therapeutic agents of the combination of the invention may be administered simultaneously or sequentially and in any order, and the components may be administered separately or as a fixed combination. For example, the pharmaceutical composition for use in a method of treating a proliferative disease according to the invention may comprise (i) administration of the first therapeutic agent (a) in free or pharmaceutically acceptable salt form, and (ii) administration of a therapeutic agent (b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual therapeutic agents of the COMBINATION OF THE INVENTION may be administered separately at different times during the course of therapy or concurrently in divided or single combination forms. The instant invention is therefore to be understood as embracing all such regimens of simultaneous or alternating treatment and the term "administering" is to be interpreted accordingly.

The effective dosage of each of the therapeutic agents employed in the COMBINATION OF THE INVENTION may vary depending on the particular compound or pharmaceutical composition employed, the mode of administration, the condition being treated, and the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single therapeutic agents required to alleviate, counter or arrest the progress of the condition.

The effective dosage of each of the therapeutic agents may require more frequent administration of one of the compound(s) as compared to the other compound(s) in the combination. Therefore, to permit appropriate dosing, packaged pharmaceutical products may contain one or more dosage forms that contain the combination of compounds, and one or more dosage forms that contain one of the combination of compounds, but not the other compound(s) of the combination.

When the therapeutic agents, which are employed in the COMBINATION OF THE INVENTION, are applied in the form as marketed as single drugs, their dosage and mode of administration can be in accordance with the information provided on the package insert of the respective marketed drug, if not mentioned herein otherwise.

The dose of COMPOUND D is preferably administered daily at a dose in the range of from about 0.05 to about 50 mg per kilogram body weight of recipient per day; preferably about 0.1-25 mg/kg/day, more preferably from about 0.5 to 10 mg/kg/day. Thus, for administration to a 70 kg person, the dosage range would most preferably be about 35-700 mg per day.

The c-Met receptor tyrosine kinase inhibitor, especially 2-fluoro-N-methyl-4-[7-quinolin-6-yl-methyl)-imidazo[1,2-b][1,2,4]triazin-2yl]benzamide, is preferably administered daily at a dose in the range of from about 0.01 mg/kg to about 100 mg/kg of body weight per day. Thus, for administration to a 70 kg person, the dosage range would most

preferably be about 0.7 mg to 7 g per day, more preferably 50 mg to 300 mg per day.

The optimal dosage of each therapeutic agent for treatment of a proliferative disease can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art.

The amount of each therapeutic agent that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone.

Frequency of dosage may vary depending on the compound used and the particular condition to be treated. In general, the use of the minimum dosage that is sufficient to provide effective therapy is preferred. Patients may generally be monitored for therapeutic effectiveness using assays suitable for the condition being treated, which will be familiar to those of ordinary skill in the art.

In a preferred embodiment, the present invention provides a pharmaceutical combination of the therapeutic agents COMPOUND D or a pharmaceuticeutically acceptable salt thereof and a c-Met receptor tyrosine kinase inhibitor, preferably 2-fluoro-N-methyl-4-[7-quinolin-6-yl-methyl)-imidazo[1,2-b][1,2,4]triazin-2yl]benzamide, or a pharmaceutically acceptable salt thereof are present in a dosage ratio in the range of 8:1 to 1:1, more preferably 4:1 to 2:1 to 1:1 respectively daily.

The optimum ratios, individual and combined dosages, and concentrations of the drug compounds that yield efficacy without toxicity are based on the kinetics of the therapeutic agents' availability to target sites, and are determined using methods known to those of skill in the art.

Moreover, the present invention provides a commercial package comprising as therapeutic agents COMBINATION OF THE INVENTION, together with instructions for the simultaneous, separate or sequential administration thereof in the treatment of a c-Met dependent proliferative disease. In a preferred embodiment, the proliferative disease is lung cancer (e.g., non-small cell lung cancer) or glioblastoma.

The following Examples illustrate the invention described above; they are not, however, intended to limit the scope of the invention in any way. The beneficial effects of the pharmaceutical combination of the present invention can also be determined by other test models known as such to the person skilled in the pertinent art.

Utility of the COMBINATION OF THE PRESENT INVENTION, as described herein, may be demonstrated in vitro, in animal test methods as well as in clinical studies.

**Reference Example 1: Combinations with COMPOUND A in Non-Small Cell Lung Cancer**

**Material and Methods**

The PI3K inhibitor 2-methyl-2-[4-(3-methyl-2-oxo-8-quinolin-3-yl-2,3-dihydro-imidazo[4,5-c]quinolin-1-yl)-phenyl]-propionitrile (COMPOUND A) and the c-MET receptor tyrosine kinase inhibitor 2-fluoro-N-methyl-4-[7-quinolin-6-yl-methyl)-imidazo[1,2-b][1,2,4]triazin-2yl]benzamide are evaluated in combination in non-small cell lung cancer models. Compound stocks of both compounds are individually prepared in DMSO. Compounds are serially diluted using a Tecan dispenser to cover a ~1000x range of concentrations. The highest concentration used for the experiment are as follows: COMPOUND A = 2.7 μM and 2-fluoro-N-methyl-4-[7-quinolin-6-yl-methyl)-imidazo[1,2-b][1,2,4]triazin-2yl]benzamide = 0.27 μM. Both single agent and combinations are tested at multiple concentrations, along with controls and one self-cross.

**Cell culture and viability measurements**

The cell lines used in this study are purchased from American Type Cell Collection, including human non small cell lung cancer cell lines EBC-1 (which bears c-Met amplification), NCI- H1993 (which bears c-MET amplified), NCI-H1648 (which bears c-Met amplification), NCI-H1573 (which bears c-Met amplification and KRAS mutation) and HCC2935 (EGFR mutation). All cell lines are maintained in their respective culture medium as specified by the provider.

For the assessment of combination effects, cells are seeded into 384-well plates at 500 cells/ well and incubated overnight. The contents of the compound master plates are pre-diluted 1:200 (1 μL compound solution to 200 μL cell RPMI-160 culture medium containing 10% fetal calf serum) before transferring 5 μL of this pre-dilution to the cell plates containing 20 μL cell culture medium, to achieve the targeted final compound concentrations as well as a vehicle (DMSO) concentration of 0.09%.

Effects of single agents as well as their combination on cell viability is assessed after 72 hours of incubation at 37 °C/5% $CO_2$ by quantification of cellular ATP levels (CellTiterGlo, Promega) using 25 μL reagent/well and n=2 replicate plates per condition. The number/viability of cells at time of compound addition is likewise assessed and used

to estimate the population doubling time of a cell line.

For untreated and treated levels $\boldsymbol{U}$ and $\boldsymbol{T}$, inhibition is calculated using the formula $\boldsymbol{I} = 1-\boldsymbol{T}/\boldsymbol{U}$, which ranges from 0% to 100% for complete inhibition. The "Growth Inhibition" $\boldsymbol{GI} = 1 - (\boldsymbol{T}-\boldsymbol{U_0})/\boldsymbol{V_0}$ when $\boldsymbol{T}<\boldsymbol{U}_0$ and $1 - (\boldsymbol{T}-\boldsymbol{U_0})/(\boldsymbol{U}-\boldsymbol{U_0})$ otherwise, where $\boldsymbol{U}_0$ is the untreated level at time zero. ***GI*** levels of 0, 100%, and 200% correspond to inactive, cytostatic, and cytotoxic compounds.

**Compound activity, synergy, and selectivity calculations**

The single agent activity for each compound is characterized by fitting a sigmoidal Hill function of the form $\boldsymbol{I} = \boldsymbol{I}_{max}C^{\alpha}/[C^{\alpha}+\boldsymbol{EC}_{50}{}^{\alpha}]$, where $C$ is the concentration, $\boldsymbol{EC}_{50}$ is the inflection point, and $\alpha$ is the sigmoidicity. The $\boldsymbol{IC}_{50}$ crossing point is calculated where the fitted curve reaches 50% inhibition.

For combinations, synergy calculations are referred to the Loewe additive model (the "drug with itself" expectation that results from adding effective doses). The Loewe expectation [Loewe S., Ergebn Physiol 27: 47-187 (1928)) is calculated at each dose pair $C_{X,Y}$ by finding the inhibition $\boldsymbol{I}_{Loewe}$ such that $(C_X/IC_X) + (C_Y/IC_Y) = 1$, and $IC_{X,Y}$ are the effective concentrations at $\boldsymbol{I}_{Loewe}$ for the fitted single agent curves, using numerical optimization [Berenbaum MC, J Theor Biol 114: 413-431 (1985)]. The "synergy score" ***S*** is calculated by averaging the difference between $\boldsymbol{I}_{data}$ and $\boldsymbol{I}_{Loewe}$ across all the tested combination dose points, excluding the highest concentration point in the dose matrix, which is most likely to be dominated by drug off-target effects. The $\boldsymbol{GI}_{max}$, is the maximum growth inhibition in the matrix (excluding the top dose pair), as a measure of overall combination effectiveness.

Selective synergy is assessed by comparing synergy scores for a combination across all tested cell lines. The "selectivity score" for that combination in a single cell line is calculated as $\boldsymbol{SS} = \text{abs}(\boldsymbol{S}) * \boldsymbol{GI}_{max} * (\boldsymbol{S} - \mu)/\sigma$, where $\mu$ is the median and $\sigma$ is the median absolute deviation of ***S*** across all cell lines. This weighted Z-score highlights combinations that are exceptionally synergistic in a cell line and that are themselves both effective and synergistic.

All calculations are performed using Chalice software (CombinatoRx, Cambridge MA). In this experiment, the Glmax data is interpreted on a scale as follows: (a) Glmax = "0" indicates no inhibition, (b) Glmax = approximately "1" indicates 100% inhibition and stasis, and (c) Glmax = 2 indicates 200% inhibition and total eradication or death of the cancer cells. Using this procedure, combination effectiveness (Glmax), excluding the highest concentration, is shown as follows:

| MaxGl | EBC-1 | H1993 | H1648 | H1573 | HCC2935 |
|---|---|---|---|---|---|
| **COMPOUND A** | 1.90 | 1.33 | 1.72 | 1.90 | 1.36 |

In this experiment, the synergy score (SS) is interpreted as follows: (a) SS = "0" indicates synergy, (b) SS = "0.1" indicates high synergy, and (c) SS = negative value of "-0.01 to -0.07" indicates additivity. The selective synergy score that is obtained for the combination, excluding the highest concentration, is shown as follows:

| SS | EBC-1 | H1993 | H1648 | H1573 | HCC2935 |
|---|---|---|---|---|---|
| **COMPOUND A** | 0.06 | 0.07 | 0.07 | 0.00 | 0.04 |

**Reference Example 2: Combinations with COMPOUND C in Non-Small Cell Lung Cancer**

Following the experimental procedure described in Example 1 above, the efficacy and synergy of the combination of PI3K inhibitor 5-(2,6-di-morpholin-4-yl-pyrimidin-4-yl)-4-trifluoromethyl-pyridin-2-ylamine (COMPOUND C) and the c-MET receptor tyrosine kinase inhibitor 2-fluoro-N-methyl-4-[7-quinolin-6-yl-methyl)-imidazo[1,2-b][1,2,4]triazin-2yl]benzamide are evaluated in combination in non-small cell lung cancer models. Compounds are serially diluted using a Tecan dispenser to cover a ~1000x range of concentrations. The highest concentration used for the experiment are as follows: COMPOUND C = 11 μM and 2-fluoro-N-methyl-4-[7-quinolin-6-yl-methyl)-imidazo[1,2-b][1,2,4]triazin-2yl]benzamide = 0.27 μM. Both single agent and combinations are tested at multiple concentrations, along with controls and one self-cross.

All calculations are performed using Chalice software (CombinatoRx, Cambridge MA). Using this procedure, combination effectiveness (Glmax), excluding the highest concentration, is shown as follows:

| MaxGl | EBC-1 | H1993 | H1648 | H1573 | HCC2935 |
|---|---|---|---|---|---|
| **COMPOUND C** | 1.83 | 1.67 | 1.78 | 1.54 | 1.37 |

Further, the selective synergy score for the combination, excluding the highest concentration, is shown as follows:

| SS | EBC-1 | H1993 | H1648 | H1573 | HCC2935 |
|---|---|---|---|---|---|
| COMPOUND C | 0.07 | 0.05 | -0.01 | -0.03 | -0.07 |

**Example 3: Combinations with COMPOUND D in Non-Small Cell Lung Cancer**

Following the experimental procedure described in Example 1 above, the efficacy and synergy of the combination of PI3K inhibitor COMPOUND D and the c-MET receptor tyrosine kinase inhibitor 2-fluoro-N-methyl-4-[7-quinolin-6-yl-methyl)-imidazo[1,2-b][1,2,4]triazin-2yl]benzamide are evaluated in combination in non-small cell lung cancer models. Compounds are serially diluted using a Tecan dispenser to cover a ~1000x range of concentrations. The highest concentration used for the experiment are as follows: COMPOUND D = 11 μM and 2-fluoro-N-methyl-4-[7-quinolin-6-yl-methyl)-imidazo[1,2-b][1,2,4]triazin-2yl]benzamide = 0.27 μM. The lowest concentration used for the experiment are as follows: COMPOUND D = 0.15 μM and 2-fluoro-N-methyl-4-[7-quinolin-6-yl-methyl)-imidazo[1,2-b][1,2,4]triazin-2yl]benzamide = 0.0003 μM. Both single agent and combinations are tested at multiple concentrations, along with controls and one self-cross.

All calculations are performed using Chalice software (CombinatoRx, Cambridge MA). Using this procedure, combination effectiveness (Glmax), excluding the highest concentration, is shown as follows:

| MaxGI | EBC-1 | H1993 | H1648 | H1573 | HCC2935 |
|---|---|---|---|---|---|
| COMPOUND D | 1.87 | 1.21 | 1.37 | 0.57 | 0.77 |

Further, the selective synergy score for the combination, excluding the highest concentration, is shown as follows:

| | EBC-1 | H1993 | H1648 | H1573 | HCC2935 |
|---|---|---|---|---|---|
| COMPOUND D | 0.12 | 0.11 | 0.16 | 0.00 | 0.00 |

**Reference Example 4: Combinations with COMPOUND C in Glioblastoma**

**Methods**

The PI3K inhibitor 5-(2,6-di-morpholin-4-yl-pyrimidin-4-yl)-4-trifluoromethyl-pyridin-2-ylamine (COMPOUND C) and the c-MET receptor tyrosine kinase inhibitor 2-fluoro-N-methyl-4-[7-quinolin-6-yl-methyl)-imidazo[1,2-b][1,2,4]triazin-2yl]benzamide are evaluated in combination in glioblastoma tumor models. COMPOUND C (10 mM) and 2-fluoro-N-methyl-4-[7-quinolin-6-yl-methyl)-imidazo[1,2-b][1,2,4]triazin-2yl]benzamide (10mM) are dissolved in DMSO, and are stored in aliquots at -20°C.

**Cell Culture and Cell Viability Assay**

Human Glioblastoma cell lines, U-87 MG (PTEN mutant and HGF expressing line), H4 (PTEN mutant and HGF expressing line), A172 (PTEN mutant line with low HGF expression level) and LN-229 (PTEN wildtype and HGF low) are purchased from American Type Cell Collection and are maintained in a humidified incubator at 37°C in 5 % $CO_2$ in recommended media. All four cell lines express detected MET at the mRNA level.

The cells are passaged twice a week and the medium is changed every 2 to 3 days. For cell viability assay, the cells are trypsinized using TryPLE Express and plated (4000 cells/well) on clear-bottom 96-well black plates (Costar, #3904) in triplicate, and are allowed to attach overnight followed 72 hours of incubation with various concentrations of inhibitor agents or agent combinations.

Cell viability is determined by measuring cellular ATP content using the CellTiter-Glo® (CTG) luminescent cell viability assay (Promega). Each single agent and combination treatment of cells is compared to controls, or cells treated with an equivalent volume of medium. An equal volume of the CTG reagents is added to each well at the end of the compound treatment and luminescence is recorded on an Envision plate reader (Perkin Elmer). Reduced and enhanced luminescent signal values (responses) are calculated relative to untreated (control) cells.

**Method for calculating the effect of combinations**

To evaluate the anti-proliferative activity of this PI3K inhibitor with this cMET inhbitor in a non-bias way, as well as to identify synergistic effect at all possible concentrations, the studies are conducted with a "dose matrix." This utilizes all possible permutations of serially-diluted single agent and the "dose matrix, consisted of the following:

- COMPOUND C, which is subjected to a 5 dose 2X serial dilution, with a high dose of 2.4μM and a low dose of approximately 150nM
- This c-MET inhibitor , which is subjected to a 5 dose 2X serial dilution, with a high dose of 1μM and a low dose of approximately 12nM

The synergistic interaction (analyzed using Chalice software [CombinatoRx, Cambridge MA]) is calculated by comparing the response from a combination to the response of the agent acting alone, against the drug-with-itself dose-additive reference model. Deviations from dose additives can be assessed numerically with a Combination Index (CI), which quantifies the overall strength of combination effect. This calculation (esentially a volume score) is as follows:

$$V_{HSA}=\Sigma_{X,Y} \ln f_X \ln f_Y (I_{data} - I_{HSA})$$

Additionally, CI is calculated between the data and the highest single-agent surface, normalized for single agent dilution factors (Lehar J et al (2009), "Synergistic drug combinations tend to improve therapeutically relevant selectivity", Nature Biotechnology 27: 659 - 66 (2009).)

**Data analysis**

Data evaluation and graph generation are performed using Microsoft Excel software, and Chalice software.

**Results**

The effect of COMPOUND C and 2-fluoro-N-methyl-4-[7-quinolin-6-yl-methyl)-imidazo[1,2-b][1,2,4]triazin-2yl]benzamide is evaluated in the "dose matrix" scheme as discussed above. All four cells are treated in 96-well format for 2 days with COMPOUND C and 2-fluoro-N-methyl-4-[7-quinolin-6-yl-methyl)-imidazo[1,2-b][1,2,4]triazin-2yl]benzamide. Cell viability is measured using the CellTiter-Glo assay and % inhibition data is displayed numerically as 6 X6 dose grid. Figures 1 to 4 herein provide a summary of the results from the foregoing procedure. Each data point represents averaged data from 3 wells, and the color spectrum also represents the level of the inhibition. The bolded rectangles highlighted the region where the combination is more efficacious than the single agents the same dose.

The percentage of inhibition over the entire dose grid is shown in Figures 1 to 4 herein. COMPOUND C is displaying concentration dependent anti-proliferative activity in all four cell lines, less active in the one cell line that is PTEN wild type (LN229) . 2-fluoro-N-methyl-4-[7-quinolin-6-yl-methyl)-imidazo[1,2-b][1,2,4]triazin-2yl]benzamide is displaying only moderate activities in the two HGF high cell lines (U87 MG and H4), completely inactive in the other two (A172 and LN229). When used together, synergy is observed both H4 and U87-MG, but not in A172 and LN229, evidenced by enhanced growth inhibition over each of the single agents in highlighted areas as well as the Isobolograms shown in Figures 1 to 4 herein.

This data suggests that this combination synergistically inhibits the growth of a subpopulation of the glioblastoma cell lines, PTEN mutant as well HGF high cell lines appears to be more sensitive to each of the single agents and the combination results in more pronounced growth inhibition.

**Reference Example 5: Combinations with COMPOUND C in Glioblastoma Xenograft Models**

In this experiment, the U-87 MG human glioblastoma xenograft model is used to assess the anti-tumor efficacy of the PI3K inhibitor 5-(2,6-di-morpholin-4-yl-pyrimidin-4-yl)-4-trifluoromethyl-pyridin-2-ylamine (COMPOUND C) and c-Met inhibitor 2-fluoro-N-methyl-4-[7-quinolin-6-yl-methyl)-imidazo[1,2-b][1,2,4]triazin-2yl]benzamide as single agents or in combination. U-87 MG tumor cells obtained from the National Cancer Institute are reported to be homozygous for likely oncogenic variants of CDKN2A, CDKN2C, CDKN2a, and PTEN; no mutations are detected in MET, PIK3CA, or 58 other genes that are frequently altered in neoplastic cells; and HGF expressing.

Mice are treated daily for 21 days with 17.7 and 35.3 mg/kg of the c-Met inhibitor and 32.7 mg/kg COMPOUND C monotherapies, and with the c-Met inhibitor / COMPOUND C dual therapy at 17.7:32.7. An c-Met inhibitor / COMPOUND C combination at the 58.8:5.4 mg/kg dose ratio was included in the study. The control mice received both vehicles, and

a standard preclinical temozolomide regimen monitored tumor model performance. The oral treatments began on Day 1 (D1) in nude mice with established subcutaneous tumors. Each animal was euthanized when its tumor reached the predetermined 2000 mm3 endpoint volume, or on D60, whichever came first. The study was terminated on D51. Short-term efficacy was determined from mean and median tumor volume changes on D13, the last day before more than 50% of the mice in any group exited the study. Overall efficacy was determined from the times required for tumors to progress to the volume endpoint, and from D51 survival and regression rates. This report describes the methods and results of the U87MG-e326 tumor growth delay experiment.

Mice

Female nude mice (*nu*/*nu,* Charles River) are 10 weeks old, and had a body weight (BW) range of 15.5-27.3 g, on D1 of the study. The animals are fed *ad libitum* water (reverse osmosis, 1 ppm Cl) and NIH 31 Modified and Irradiated Lab Diet® consisting of 18.0% crude protein, 5.0% crude fat, and 5.0% crude fiber. The mice are housed on irradiated Enrich-o'cobs™ Laboratory Animal Bedding in static microisolators on a 12- hour light cycle at 20-22 °C (68-72 °F) and 40-60% humidity.

**Tumor Implantation and Measurement**

The U-87 MG tumor line is obtained from the American Type Culture Collection and maintained by serial engraftment in nude mice. A tumor fragment (1 $mm^3$) is implanted subcutaneously into the right flank of each test mouse. Tumors are calipered in two dimensions to monitor growth as their mean volume approached the desired 100-150 $mm^3$ range. Tumor size, in $mm^3$, was calculated from: Tumor Volume = (*width*$^2$ *x length)*/ *2,* where width and length (in mm) are measurements of the tumor. Tumor weight can be estimated with the assumption that 1 mg is equivalent to 1 $mm^3$ of tumor volume. Fourteen days after tumor cell implantation, on D1 of the study, mice with individual tumor volumes of 63-196 $mm^3$ are sorted into seven groups, with group mean tumor volumes of 122-127 $mm^3$.

**Test Articles**

The c-Met inhibitor 2-fluoro-N-methyl-4-[7-quinolin-6-yl-methyl)-imidazo[1,2-b][1,2,4]triazin-2yl]benzamide (di-HCl salt, 84.98% free base) is stored at 4 °C and is added to deionized water at 2X the final concentration and sonicated until dissolved. An equal volume of 0.5% methylcellulose and 0.1% Tween® 80 in deionized water is added to provide a 5.884 mg/mL dosing solution in 0.25% methylcellulose and 0.05% Tween® 80 in deionized water (Vehicle 1). Portions of this solution are diluted with Vehicle 1 to provide 3.53 and 1.765 mg/mL dosing solutions.

COMPOUND C (HCl salt, 91.85% free base) is stored at -20 °C and is added to 1% Tween® 80 in deionized water and sonicated to obtain a uniform suspension at 2X the final concentration. An equal volume of 1% methylcellulose in deionized water is added, and the mixture is stirred and sonicated to obtain an opaque colorless 3.266 mg/mL dosing suspension in 0.5% methylcellulose and 0.5% Tween® 80 in deionized water (Vehicle 2). The 0.544 mg/mL dosing suspension is obtained by diluting a portion of the first suspension with Vehicle 2.

Temozolomide (Temodar®, Schering Corporation, 100 mg capsule, Lot# IRSA001) is prepared once, by suspension in deionized water, and stored at 4 °C.

**Treatment Plan**

Both test agents and the vehicles are administered by oral gavage (p.o.) once daily for twenty-one consecutive days (qd x 21). In combination therapies, 2-methyl-2-[4-(3-methyl-2-oxo-8-quinolin-3-yl-2,3-dihydro-imidazo[4,5-c]quinolin-1-yl)-phenyl]-propionitrile (COMPOUND A) is dosed within 60 minutes after the c-Met inhibitor. Temozolomide is administered p.o. once daily for five consecutive days (qd x 5). In all groups, the dosing volume of 10 mL/kg (0.2 mL/20 g mouse) is scaled to the weight of each animal as determined on the day of dosing, except on weekends when the previous BW is carried forward.

Seven groups of nude mice (n = 10/group) are treated as follows. Group 1 mice receive Vehicles 1 and 2, and served as controls for all analyses. On D7, this group is inadvertently dosed with an IgG negative control antibody; it was concluded that this error had no impact on control tumor growth. Groups 2 and 3 receive the c-Met monotherapy monotherapies at 17.7 and 35.3 mg/kg (equivalent to 15 and 30 mg/kg free base), respectively. Group 4 received COMPOUND C monotherapy at 32.7 mg/kg (30 mg/kg free base). Group 5 received 17.7 mg/kg the c-Met inhibitor in combination with 32.7 mg/kg COMPOUND C. Group 6 received 58.8 mg/kg the c-Met inhibitor in combination with 5.4 mg/kg COMPOUND C (equivalent to 50 and 5 mg/kg free base, respectively). Group 7, the reference group, received temozolomide monotherapy at 100 mg/kg.

**Tumor Growth Inhibition**

Short-term efficacy is determined on D13. Prior to D13, one Group 1 animal with a tumor doublet is exited when the sum of its tumor volumes progressed to the endpoint. The last recorded tumor volume for this animal is included in the D13 data. $\Delta TV$, the difference in tumor volume between D1 (the start of dosing) and the endpoint day, was determined for each animal. For each treatment group, the response on the endpoint day was calculated by one of the following relations:

$$T/C\ (\%) = 100 \times \Delta T/\Delta C, \text{ for } \Delta T > 0$$

$$T/T0\ (\%) = 100 \times \Delta T/T0, \text{ for } \Delta T < 0,$$

where

$\Delta T$ = (mean tumor volume of the treated group on the endpoint day) - (mean tumor volume of the treated group on D1),
$\Delta C$ = (mean tumor volume of the control group on the endpoint day) - (mean tumor volume of the control group on D1), and
T0 = mean tumor volume of the treated group on D1.

A negative T/T0 value represents net tumor reduction for a group. A T/C value of 40% or less suggests potential therapeutic activity.

**Tumor Growth Delay**

Each animal is euthanized when its neoplasm reached the endpoint volume (2000 mm3), or on the last day of the study (D51). For each animal whose tumor reaches the endpoint volume, the time to endpoint (TTE) was calculated by the following equation:

$$TTE = (\log_{10}(\text{endpoint volume}) - b)/\ m$$

where TTE is expressed in days, endpoint volume is in mm3, b is the intercept, and m is the slope of the line obtained by linear regression of a log-transformed tumor growth data set. The data set is comprised of the first observation that exceeds the study endpoint volume and the three consecutive observations that immediately precede the attainment of the endpoint volume. Any animal with a tumor that does not reach the endpoint is assigned a TTE value equal to the last day of the study. Any animal classified as having died from TR causes, is assigned a TTE value equal to the day of death. Any animal classified as having died from NTR causes (other than metastasis) is excluded from TTE calculations.

Treatment efficacy is determined from tumor growth delay (TGD), which is defined as the increase in the median TTE for a treatment group compared to the control group:

$$TGD = T - C,$$

expressed in days, or as a percentage of the median TTE of the control group:

$$\%\ TGD = [(T\text{-}C)\ C] \quad \times 100$$

where:

T = median TTE for a treatment group,

C = median TTE for the designated control group.

**MTV and Criteria for Regression Responses**

Treatment efficacy may also be determined from the tumor volumes of animals remaining in the study on the last day, and from the number of regression responses. The MTV(n) is defined as the median tumor volume on D51 in the number of animals remaining, n, whose tumors had not attained the endpoint volume. Treatment may cause a partial regression (PR) or a complete regression (CR) of the tumor in an animal. A PR indicates that the tumor volume is 50% or less of its D1 volume for three consecutive measurements during the course of the study, and equal to or greater than 13.5 $mm^3$ for one or more of these three measurements. A CR indicates that the tumor volume is less than 13.5 $mm^3$ for three consecutive measurements during the course of the study. Any animal that presented with a CR response on the last day of the study is additionally classified as a tumor-free survivor (TFS).

**Toxicity**

Animals are weighed on Days 1-5, on each treatment day (except weekends), and twice weekly thereafter, until the end of the study. Group mean BW nadir determinations exclude measurements taken after more than 50% of the assessable mice in the group had exited the study, and also excluded any animal that was classified as an NTR death during the study. Acceptable toxicity for the maximum tolerated dose (MTD) is defined as a group mean BW loss of less than 15% during the test, and not more than one TR death among ten animals. Any animal with BW losses exceeding 15% for three consecutive measurements, or with a BW loss exceeding 20% for one measurement, are euthanized, and classified as a TR death unless it was the first death in the group. A death is classified as Non-Treatment Related (NTR) if there was no evidence that the death is related to treatment side effects and the death occurs more than 14 days after dosing ended. NTR deaths are categorized as NTRa (due to accident or error), NTRu (due to unknown causes), or NTRm (necropsy-confirmed tumor dissemination by invasion and/or metastasis). To conserve animals while providing maximum information, the first death in a group was classified as NTRu; such an NTR death was to be reclassified as TR if two subsequent TR deaths are recorded in the same group.

**Statistical and Graphic Analyses**

All statistical and graphic analyses are performed with Prism 3.03 (GraphPad) for Windows. The mean tumor volume changes for Groups 1-7 are first compared with analysis of variance (ANOVA), with Bartlett's test. When Bartlett's test indicates significant differences among variances ($P < 0.0001$), the groups are compared with Kruskal-Wallis analysis, which show significant differences among median tumor volume changes ($P < 0.0001$). A post hoc Dunn's multiple comparison test compares the drug-treated groups to control Group 1. These non-parametric tests are repeated once to compare one of the combination therapy groups to its corresponding monotherapies.

Survival is analyzed by the Kaplan-Meier method. The logrank (Mantel-Cox) test is employed to analyze the significance of the difference between the overall survival experiences (survival curves) of two groups. The logrank test analyzes the individual TTEs for all animals in a group, except those excluded as non-treatment related (NTR) deaths. The two-tailed statistical analyses are conducted at $P = 0.05$. Prism summarizes test results as not significant (ns) at $P > 0.05$, significant (symbolized by "*") at $0.01 < P \leq 0.05$, very significant ("**") at $0.001 < P \leq 0.01$, and extremely significant ("***") at $P \leq 0.001$. Because tests of statistical significance do not provide an estimate of the magnitude of the difference between groups, all levels of significance are described as either significant or not significant within the text of this report.

<u>Results</u>

Following the above procedure, the following results are obtained in the study:

| Grp | n | Treatment | Dose (mg/kg) | T/C or T/T0 | Statistical Signif. (Dunn's) | Median TTE | % TGD | Statistical Signif. (Logrank) | Regress. (Deaths) |
|---|---|---|---|---|---|---|---|---|---|
| 1 | 10 | Veh. 1<br>Veh. 2 | | -- | vs. G1:--<br>vs. G2:--<br>vs. G4:-- | 11.7 | -- | vs. G1: --<br>vs. G2: --<br>vs. G4: --<br>vs. G5: -- | 0<br>(0) |
| 2 | 10 | c-Met inh. | 17.7 | 5% | vs. G1: <0.01 | 33.1 | 183 | vs. G1: *** | 4 PR<br>(0) |

(continued)

| Grp | n | Treatment | Dose (mg/kg) | T/C or T/T0 | Statistical Signif. (Dunn's) | Median TTE | % TGD | Statistical Signif. (Logrank) | Regress. (Deaths) |
|---|---|---|---|---|---|---|---|---|---|
| | | | | | vs. G2:--<br>vs. G4:-- | | | vs. G2: --<br>vs. G4: --<br>vs. G5: -- | |
| 3 | 10 | c-Met inh. | 35.3 | 1% | vs. G1: <0.001<br>vs. G2:--<br>vs. G4:-- | 35.1 | 200 | vs. G1: ***<br>vs. G2: ns<br>vs. G4: --<br>vs. G5: -- | 4 PR<br>(0) |
| 4 | 9 | CMPD. C | 32.7 | 31% | vs. G1: ns<br>vs. G2:--<br>vs. G4:-- | 21.0 | 79 | vs. G1: ***<br>vs. G2: --<br>vs. G4: --<br>vs. G5: -- | 0<br>(1 TR, 1 NTR) |
| 5 | 9 | c-Met | 17.7 | -83% | vs. G1: | 40.4 | 245 | vs. G1: | 6 PR, |
| | | inh.<br>CMPD. C | 32.7 | | <0.001<br>vs. G2:<0.05<br>vs. G4:<0.001 | | | ***<br>vs. G2: ns<br>vs. G4: ***<br>vs. G5: -- | 3 CR<br>(1 NTR) |
| 6 | 10 | c-Met inh.<br>CMPD. C | 58.8<br>5.4 | -75% | vs. G1: <0.001<br>vs. G2:--<br>vs. G4:-- | 51.0 | 336 | vs. G1: ***<br>vs. G2: --<br>vs. G4: --<br>vs. G5: *** | 3 PR,<br>7 CR<br>5 TFS<br>(0) |
| 7 | 10 | Temozol omide | 100 | 48% | vs. G1:ns<br>vs. G2:--<br>vs. G4:-- | 17.7 | 51 | vs. G1: ***<br>vs. G2: --<br>vs. G4: --<br>vs. G5: -- | 0<br>(0) |

Study Endpoint = 2000 $mm^3$, Short term activity = 13 days, Days in progression 51.
N = number of animals in a group not dead from accidental or unknown causes, or euthanized for sampling.
**T/C = 100 x ($\Delta$T/$\Delta$C)** = % change between D1 and D13 in mean tumor volume of treated group compared with control group.
**$T/T_0$= 100 x ($\Delta$T/T0)** = % change between D1 and D13 in mean tumor volume of treated group compared with its initial volume, when $\Delta T = 0$.
**Statistical Significance (Dunn's test)** = P value from Kruskal-Wallis Dunn's multiple comparison test: ns = not significant = P > 0.05, compared to indicated group
**Statistical Significance (Logrank test)** = P value from logrank test compared to indicated group: ns = not significant; results are deemed significant at $P \leq 0.05$.

The c-Met inhibitor monotherapies at 15 mg/kg and 30 mg/kg resulted in 5% and 1% T/C respectively. COMPOUND C at 30 mg/kg results in T/C of 31%. Combination of the c-Met inhibitor 15 mg/kg with COMPOUND C at 30 mg/kg achieves tumor regression with T/T0: -83%, which is significant better than either single agent alone.

After termination of treatment, percent tumor growth delay (%TGD) is evaluated by monitor tumor growth and record time to end point (TTE). Vehicle-treated group reached end point at 11.7 days. The c-Met inhibitor at 15 mg/kg and 30 mg/kg increases median TTE by 21.4 days (183%TGD) and 23.4 days (200%TGD) respectively. COMPOUND C at 30 mg/kg increased median TTE by 9.3 days (79%TGD). Combination of c-Met inhibitor at 15 mg/kg with COMPOUND C at 30 mg/kg demonstrated median TTE by 28.7 days, which is 245% TGD.

**Example 6: Combinations with COMPOUND D in Non-Small Cell Lung Cancer Xenograft Models**

In this experiment, the NCI-H1993 human non-small cell lung carcinoma xenograft model is used to assess the anti-tumor efficacy of the PI3K inhibitor COMPOUND D and c-Met inhibitor 2-fluoro-N-methyl-4-[7-quinolin-6-yl-methyl)-imidazo[1,2-b][1,2,4]triazin-2yl]benzamide as single agents or in combination. NCI-H1993 tumor cells obtained from the American Type Culture Collection (ATCC) are derived from a lymph node metastasis of a patient having a stage 3A lung adenocarcinoma and a history of smoking.

Mice

Female athymic nude mice (Crl:NU (Ncr)-*Foxn1$^{nu}$,* Charles River) are 8 weeks old, and have a body weight (BW) range of 16.1-26.2 g, on D1 of the study. The animals are fed *ad libitum* water (reverse osmosis, 1 ppm Cl) and NIH 31 Modified and Irradiated Lab Diet® consisting of 18.0% crude protein, 5.0% crude fat, and 5.0% crude fiber. The mice are housed on irradiated Enrich-o'cobs™ Laboratory Animal Bedding in static microisolators on a 12- hour light cycle at 20-22 °C (68-72 °F) and 40-60% humidity.

**Tumor Implantation and Measurement**

The NCI-H1993 tumor line is obtained from the American Type Culture Collection and maintained at DRS_NC in RPMI-1640 medium containing 100 unites/ penicillin G sodium, 100 μg/mL streptomycin sulfate, 25 μg/mL gentamicin, 10% fetal bovine serum, and 2 mM glutamine. The tumor cells are cultured in tissue culture flasks in a humidified incubator at 37°C in an atmosphere of 5% $CO_2$ and 95% air.

The tumor cells used for implimitentation are harvested during log phase growth and resuspended in cold PBS containing 50% Matrigel™ (BD Biosciences). Each mouse is injected subcutaneously in the right flank with 1 x $10^7$ cells (0.2 mL cell suspension). Tumors are calipered in two dimensions to monitor growth as their mean volume approached the desired 200-250 $mm^3$ range. Tumor size, in $mm^3$, was calculated from: Tumor Volume = (*$width^2$ x length)*/2, where width and length (in mm) are measurements of the tumor. Tumor weight can be estimated with the assumption that 1 mg is equivalent to 1 $mm^3$ of tumor volume. Fourteen days after tumor cell implantation, on D1 of the study, mice with individual tumor volumes of 172-256 $mm^3$ are sorted into groups of ten mice, with group mean tumor volumes of 206-215 $mm^3$.

**Test Articles**

The c-Met inhibitor 2-fluoro-N-methyl-4-[7-quinolin-6-yl-methyl)-imidazo[1,2-b][1,2,4]triazin-2yl]benzamide (HCl salt, 84.98% free base) is stored at 4 °C and is added to a volume of deionized water equal to 1/2 of the final volume and sonicated until dissolved to obtain a uniform 2X suspension. An equal volume of 0.5% methylcellulose: 0.1% Tween® 80 is added to provide a 1.77 mg/mL solution with final vehicle concentrations of 0.25% methylcellulose and 0.05% Tween® 80 in deionized water (Vehicle 1). Fresh solutions are prepared once daily for the p.m. dosing and stored at room temperature until the a.m. dosing.

COMPOUND D is suspended at 3.0 mg/mL in 0.5% methylcellulose in deionized water. The vehicle is added in portions equal to 1/3 of the final volume. The suspension is vortexed after each addition, and then finally probe sonicated on ice to prepare a homogeneous white suspension. A fresh suspension is prepared weekly, stored at 4°C, and re-suspended prior to dosing.

Vehicle 2 is prepared at a final vehicle concentration of 35% D5W (5% dextrose in water): 5% of 10% Tween® 80 (0.05% final concentration): 60% 100 mM acetate buffer pH 4.5.

**Treatment Plan**

The c-MET inhibitor and the vehicle is each administered via oral gavage (p.o.) twice daily for the duration of the study (b.i.d. to end). COMPOUND D is administered via oral gavage (p.o.) once daily for the duration of the study (qd to end). In combination therapies, COMPOUND D is dosed within 30 minutes after the c-Met inhibitor. In all groups, the dosing volume of 10 mL/kg (0.2 mL/20 g mouse) is scaled to the weight of each animal as determined on the day

of dosing, except on weekends when the previous BW is carried forward.

The groups of nude mice (n = 10/group) are treated as follows. Group 1 receive Vehicle 1 and Vehicle 2 and serve as controls for the study. Group 2 receives 17.7 mg/kg of the c-MET inhibitor monotherapy twice daily for the duration of the study (b.i.d. to end). Group 3 receives 30 mg/kg COMPOUND D monotherapy once daily for the duration of the study (qd to end). Group 4 receives 17.7 mg/kg of the c-MET inhibitor b.i.d. to end in combination with 30 mg/kg COMPOUND D qd to end.

**Tumor Growth Inhibition**

Short-term efficacy is determined on Day 20 (D20). Prior to D20, one animal in Group 1 and one animal in Group 3 had exited for tumor progression; the final tumor volume of each is carried forward and included in the group mean volume.

$\Delta$TV, the difference in tumor volume between D1 (the start of dosing) and the endpoint day, was determined for each animal. For each treatment group, the response on the endpoint day was calculated by one of the following relations:

$$T/C\ (\%) = 100 \times \Delta T/\Delta C, \text{ for } \Delta T > 0$$

$$T/T0\ (\%) = 100 \times \Delta T/T0, \text{ for } \Delta T < 0,$$

where

$\Delta$T = (mean tumor volume of the treated group on the endpoint day) - (mean tumor volume of the treated group on D1),
$\Delta$C = (mean tumor volume of the control group on the endpoint day) - (mean tumor volume of the control group on D1), and
T0 = mean tumor volume of the treated group on D1.

A negative T/T0 value represents net tumor reduction for a group. A T/C value of 40% or less suggests potential therapeutic activity.

**Tumor Growth Delay**

Tumors are calipered twice weekly, and each animal is euthanized when its neoplasm reached the endpoint volume (600 $mm^3$), or on the last day of the study (D71), whichever comes first. The time to endpoint (TTE) for each mouse is calculated by the following equation:

$$TTE = (\log_{10} (\text{endpoint volume}) - b)/ m$$

where TTE is expressed in days, endpoint volume is in $mm^3$, b is the intercept, and m is the slope of the line obtained by linear regression of a log-transformed tumor growth data set. Each data set is comprised of the first observation that exceeds the study endpoint volume and the three consecutive observations that immediately precede the attainment of the endpoint volume. Any animal with a tumor that does not reach the endpoint is assigned a TTE value equal to the last day of the study. Any animal classified as having died from treatment-related (TR) causes, is assigned a TTE value equal to the day of death. Any animal classified as having died from non-treatment related (NTR) causes (other than metastasis) is excluded from TTE calculations.

Treatment efficacy is determined from tumor growth delay (TGD), which is defined as the increase in the median TTE for a treatment group compared to the control group:

$$TGD = T - C,$$

expressed in days, or as a percentage of the median TTE of the control group:

$$\%\ TGD = [(T\text{-}C)\ C] \quad \times 100$$

where:

T = median TTE for a treatment group,

C = median TTE for the designated control group.

**MTV and Criteria for Regression Responses**

Treatment efficacy may also be determined from the tumor volumes of animals remaining in the study on the last day, and from the number of regression responses. The MTV(n) is defined as the median tumor volume on D71 in the number of animals remaining, n, whose tumors had not attained the endpoint volume.

Treatment may cause a partial regression (PR) or a complete regression (CR) of the tumor in an animal. A PR indicates that the tumor volume is 50% or less of its D1 volume for three consecutive measurements during the course of the study, and equal to or greater than 13.5 $mm^3$ for one or more of these three measurements. A CR indicates that the tumor volume is less than 13.5 $mm^3$ for three consecutive measurements during the course of the study.

**Toxicity**

Animals are weighed on Days 1-5, on each treatment day (except weekends), and twice weekly thereafter, until the end of the study. The maximum group mean body weight (BW) loss is determined for the interval between D1 and D20 as well as for the entire study. Group mean BW nadir determinations exclude measurements taken after more than 50% of the assessable mice in the group have exited the study, and also exclude any animal that was classified as a non-treatment related (NTR) death during the study. Acceptable toxicity for the maximum tolerated dose (MTD) is defined as a group mean BW loss of less than 15% during the test, and not more than one TR death among ten animals. Any animal with BW losses exceeding 15% for three consecutive measurements, or with a BW loss exceeding 20% for one measurement, are euthanized, and classified as a TR death unless it was the first death in the group. A death is classified as NTR if there was no evidence that the death is related to treatment side effects and the death occurs more than 14 days after dosing ended. NTR deaths are categorized as NTRa (due to accident or error), NTRu (due to unknown causes), or NTRm (necropsy-confirmed tumor dissemination by invasion and/or metastasis). To conserve animals while providing maximum information, the first death in a group was classified as NTRu; such an NTR death was to be reclassified as TR if two subsequent TR deaths are recorded in the same group.

**Statistical and Graphic Analyses**

Statistical and graphic analyses are performed with Prism 3.03 (GraphPad) for Windows. The mean tumor volume changes for all groups are compared with one-way analysis of variance (ANOVA), with Bartlett's test for equal variance. Because differences among the variances are significant ($P < 0.01$), the groups ar compared with Kruskal-Wallis analysis and Dunn's post hoc multiple comparison test. These latter tests are repeated to compare the combination with its component monotherapies.

Survival is analyzed by the Kaplan-Meier method based on TTE values. The logrank test is employed to determine the significance of the difference between the overall survival experiences (survival curves) of two groups. The two-tailed statistical analyses are conducted at P = 0.05. Prism summarizes test results as not significant (ns) at $P > 0.05$, significant (symbolized by "*") at $0.01 < P \leq 0.05$, very significant ("**") at $0.001 < P \leq 0.01$, and extremely significant ("***") at $P \leq 0.001$. Because tests of statistical significance do not provide an estimate of the magnitude of the difference between groups, all levels of significance are described as either significant or not significant within the text of this report.

Results

Following the above procedure, the following results are obtained at the end of the study as defined above:

| Grp | n | Treatment | Median TTE | T-C | % TGD | Statistical Signif. (Logrank) | T/C or T/T0 | Statistical Signif. (Dunn's) | Mean body weight Nadir | Regression (Deaths) |
|---|---|---|---|---|---|---|---|---|---|---|
| 1 | 10 | Veh. 1<br>Veh. 2 | 25.0 | -- | -- | vs. G1: --<br>vs. G2: --<br>vs. G3: -- | -- | vs. G1:--<br>vs. G2:--<br>vs. G3:-- | -- | 0<br>(0) |
| 2 | 9 | c-Met<br>in hibitor | 49.2 | 24.2 | 97 | vs. G1:<br>0.0002<br>vs. G2: --<br>vs. G3: -- | 15% | vs. G1: ns<br>vs. G2:--<br>vs. G3:-- | -- | 1 PR<br>(1 NTR) |
| 3 | 10 | CMPD. D | 24.0 | -1.1 | -4 | vs. G1:<br>ns<br>vs. G2: --<br>vs. G3: -- | 117% | vs. G1: ns<br>vs. G2:--<br>vs. G3:-- | -- | 1 PR<br>(0) |
| 4 | 9 | c-Met<br>in hibitor<br>and<br>CMPD. D | 71.0 | 46 | 184 | vs. G1:<br><0.0001<br>vs. G2:<br>0.0344<br>vs. G3:<br>0.006 | -17% | vs. G1:<br><0.01<br>vs. G2: ns<br>vs. G3:<br><0.001 | -1.3%<br>Day 51 | 1 PR<br>(1 TR, 1 NTR) |

**N** = number of animals in a group not dead from treatment-related, accidental, or unknown causes, or euthanized for sampling
**T/C = 100 x (ΔT/ΔC)** = % change between D1 and D20 in mean tumor volume of treated group compared with control group.
**$T/T_0$= 100 x (ΔT/T0)** = % change between D1 and D20 in mean tumor volume of treated group compared with its initial volume, when ΔT = 0.
**Statistical Significance (Dunn's test)** = P value from Kruskal-Wallis Dunn's multiple comparison test: ns = not significant = P > 0.05, compared to indicated group
**Statistical Significance (Logrank test)** = P value from logrank test compared to indicated group: ns = not significant; results are deemed significant at P ≤ 0.05.

The c-Met inhibitor monotherapy at 17.7 mg/kg p.o bid to end does not achieve significant inhibition relative to control (Group 1) due to variable response and overlapping change in tumor volume ranges. COMPOUND D monotherapy at 30 mg/kg p.o. qd to end results in non-significant median tumor growth acceleration. Combination of the c-Met inhibitor and COMPOUND D results in -17% $T/T_0$ and significant median activites (P < 0.01, Dunnett's test), but does not improve significantly upon c-Met inhibitor monotherapy on Day 20.

Overall, c-Met inhibitor monotherapy increases median TTE by 97% and significantly extends survival (P<0.001, logrank test), yielding four D71 survivors and one PR. Well-tolerated COMPOUND D monotherapy results in non-significantly briefer overall survival than control (Group 1).

Combination of the c-Met inhibitor and COMPOUND D increases median TTE by the maximum possible 184%. The combination group has 8 survivors and 1 partial regression. The combination improves significantly upon both monotherapies. Figure 5 herein provides a summary of the results.

In summary, the c-Met inhibitor monotherapy at 17.7 mg/kg b.i.d. to end produces 15% T/C and non-significant D20 tumor growth inhibition, but yieldes a 97% increase in median TTE and significant survival extension. COMPOUND D monotherapy at 30 mg/kg qd to end is not significantly active (inactive). The combination displays favorable drug interactions by causing tumor reduction on D20 and producing the maximm possible 184% increase in median TTE. Significant improvements over c-Met inhibitor monotherapy are not observed on D20; however, the combination significantly extends survival with respect to both corresponding monotherapies. One death during c-Met inhibitor monotherapy and 2 deaths during combination therapy are due to individual body weight losses. As group mean body weight losses is zero or negligible, it is uncertain whether the c-Met inhibitor monotherapy is at the maximum terminal dose, and whether the combination exceeds the maximum terminal dose for the model.

## Claims

1. A pharmaceutical combination comprising (a) (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or a pharmaceutically acceptable salt thereof, and (b) at least one c-Met receptor tyrosine kinase inhibitor or pharmaceutically acceptable salt thereof, for use in the treatment of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation.

2. The pharmaceutical combination for use according to claim 1, wherein the c-Met receptor tyrosine kinase inhibitor is 2-fluoro-N-methyl-4-[7-quinolin-6-yl-methyl)-imidazo[1,2-b][1,2,4]triazin-2yl]benzamide, or a pharmaceutically acceptable salt thereof.

3. The pharmaceutical combination for use according to claim 1 or claim 2, wherein the proliferative disease is cancer.

4. The pharmaceutical combination for use according to any one of claims 1 to 3, wherein the proliferative disease is a cancer selected from the group consisting of benign and malignant tumors of the breast, bladder, cervix, cholangiocarcinoma, colorectum, esophagus, gastric, head and neck, kidney, liver, lung, nasopharygeal, ovary, pancreas, prostate, thyroid, endometrial, sarcomas of the musculoskeletal system, sarcomas of soft tissues sarcomas, multiple myeloma, lymphomas, adult T cell leukemia, acute myelogenous leukemia, chronic myeloid leukemia, glioblastomas, astrocytomas, melanoma, mesothelioma and Wilm's tumor.

5. The pharmaceutical combination for use according to any one of claims 1 to 4, wherein the proliferative disease is lung cancer (e.g., non-small cell lung cancer) or glioblastoma.

6. The pharmaceutical combination for use according to any one of claims 1 to 5, wherein the administration of the combination is simultaneous, separate or sequential.

7. A pharmaceutical composition comprising a pharmaceutical combination according to claim 1 or claim 2 and at least one pharmaceutically acceptable carrier.

8. The pharmaceutical combination for use according to claim 1 in a method of inhibiting the formation of metastases in a subject having a cancer which comprises administering to a subject in need thereof a pharmaceutically effective amount of a pharmaceutical combination according to claim 1.

9. The pharmaceutical combination for use in a method according to claim 8, wherein the proliferative disease or

cancer is a cancer selected from the group consisting of benign and malignant tumors of the breast, bladder, cervix, cholangiocarcinoma, colorectum, esophagus, gastric, head and neck, kidney, liver, lung, nasopharygeal, ovary, pancreas, prostate, thyroid, endometrial, sarcomas of the musculoskeletal system, sarcomas of soft tissues sarcomas, multiple myeloma, lymphomas, adult T cell leukemia, acute myelogenous leukemia, chronic myeloid leukemia, glioblastomas, astrocytomas, melanoma, mesothelioma and Wilm's tumor.

**10.** The pharmaceutical combination for use in a method according to claim 9, wherein the treatment comprises administering the amount of therapeutic agent (a) and the amount of therapeutic agent (b) separately or sequentially.

**11.** A pharmaceutical combination comprising (*S*)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or a pharmaceutically acceptable salt thereof, and a c-Met receptor tyrosine kinase inhibitor 2-fluoro-N-methyl-4-[7-quinolin-6-yl-methyl)-imidazo[1,2-b][1,2,4]triazin-2yl]benzamide, or a pharmaceutically acceptable salt thereof.

**12.** (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or a pharmaceutically acceptable salt thereof, for use in the treatment of proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with at least one c-Met receptor tyrosine kinase inhibitor, or a pharmaceutically acceptable salt thereof.

**13.** The compound for use according to claim 12, wherein the c-Met receptor tyrosine kinase inhibitor is 2-fluoro-N-methyl-4-[7-quinolin-6-yl-methyl)-imidazo[1,2-b][1,2,4]triazin-2yl]benzamide, or a pharmaceutically acceptable salt thereof.

**14.** A c-Met receptor tyrosine kinase inhibitor which is 2-fluoro-N-methyl-4-[7-quinolin-6-yl-methyl)-imidazo[1,2-b][1,2,4]triazin-2yl]benzamide, or a pharmaceutically acceptable salt thereof, for use in the treatment of a proliferative disease having a dysregulation of c-Met and/or the HGF/c-Met signaling pathway by HGF/ c-Met gene overexpression or amplification, HGF-dependent autocrine and paracrine activation, and/or c-Met receptor activation through a genetic mutation, in combination with (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND D), or a pharmaceutically acceptable salt thereof.

## Patentansprüche

**1.** Pharmazeutische Zusammensetzung, umfassend (a) (*S*)-Pyrrolidin-1,2-dicarbonsäure-2-amid-1-({4-methyl-5-[2-(2,2,2-trifluor-1,1-dimethylethyl)-pyridin-4-yl]thiazol-2-yl}amid) (VERBINDUNG D) oder ein pharmazeutisch unbedenkliches Salz davon und (b) mindestens einen c-Met-Rezeptortyrosinkinase-Inhibitor oder ein pharmazeutisch unbedenkliches Salz davon, zur Verwendung bei der Behandlung einer proliferativen Krankheit mit Dysregulation von c-Met und/oder dem HGF/c-Met-Signalweg durch HGF/c-Met-Gen-Überexpression oder -Amplifikation, HGF-abhängiger autokriner und parakriner Aktivierung und/oder c-Met-Rezeptor-Aktivierung durch eine genetische Mutation.

**2.** Pharmazeutische Kombination zur Verwendung nach Anspruch 1, wobei es sich bei dem c-Met-Rezeptortyrosinkinase-Inhibitor um 2-Fluor-N-methyl-4-[7-chinolin-6-yl-methyl)imidazo[1,2-b]-[1,2,4]triazin-2-yl]benzamid oder ein pharmazeutisch unbedenkliches Salz davon handelt.

**3.** Pharmazeutische Kombination zur Verwendung nach Anspruch 1 oder Anspruch 2, wobei es sich bei der proliferativen Krankheit um Krebs handelt.

**4.** Pharmazeutische Kombination zur Verwendung nach einem der Ansprüche 1 bis 3, wobei es sich bei der proliferativen Krankheit um eine Krebserkrankung aus der Gruppe bestehend aus gutartigen und bösartigen Tumoren der Brust, der Blase, des Gebärmutterhalses, Cholangiokarzinom, des Kolorektums, der Speiseröhre, des Magens, des Kopfes und Halses, der Niere, der Leber, der Lunge, des Nasenrachenraums, des Eierstocks, der Bauchspeicheldrüse, der Prostata, der Schilddrüse, des Endometriums, Sarkomen des Stütz- und Bewegungsapparats, Sarkomen von Weichteilsarkomen, multiplem Myelom, Lymphomen, adulter T-Zell-Leukämie, akuter myeloischer Leukämie, chronischer myeloischer Leukämie, Glioblastomen, Astrozytomen, Melanom, Mesotheliom und Wilms-Tumor handelt.

5. Pharmazeutische Kombination zur Verwendung nach einem der Ansprüche 1 bis 4, wobei es sich bei der proliferativen Krankheit um Lungenkrebs (z. B. nichtkleinzelligen Lungenkrebs) oder Glioblastom handelt.

6. Pharmazeutische Kombination zur Verwendung nach einem der Ansprüche 1 bis 5, wobei die Kombination gleichzeitig, separat oder aufeinanderfolgend verabreicht wird.

7. Pharmazeutische Zusammensetzung, umfassend eine pharmazeutische Kombination nach Anspruch 1 oder Anspruch 2 und mindestens einen pharmazeutisch unbedenklichen Träger.

8. Pharmazeutische Kombination zur Verwendung nach Anspruch 1 bei einem Verfahren zur Inhibierung der Bildung von Metastasen bei einem Individuum mit einer Krebserkrankung, bei dem man einem Individuum, bei dem diesbezüglicher Bedarf besteht, eine pharmazeutisch wirksame Menge einer pharmazeutischen Kombination nach Anspruch 1 verabreicht.

9. Pharmazeutische Kombination zur Verwendung bei einem Verfahren nach Anspruch 8, wobei es sich bei der proliferativen Erkrankung bzw. Krebserkrankung um eine Krebserkrankung aus der Gruppe bestehend aus gutartigen und bösartigen Tumoren der Brust, der Blase, des Gebärmutterhalses, Cholangiokarzinom, des Kolorektums, der Speiseröhre, des Magens, des Kopfes und Halses, der Niere, der Leber, der Lunge, des Nasenrachenraums, des Eierstocks, der Bauchspeicheldrüse, der Prostata, der Schilddrüse, des Endometriums, Sarkomen des Stütz- und Bewegungsapparats, Sarkomen von Weichteilsarkomen, multiplem Myelom, Lymphomen, adulter T-Zell-Leukämie, akuter myeloischer Leukämie, chronischer myeloischer Leukämie, Glioblastomen, Astrozytomen, Melanom, Mesotheliom und Wilms-Tumor handelt.

10. Pharmazeutische Kombination zur Verwendung bei einem Verfahren nach Anspruch 9, wobei die Behandlung die separate oder aufeinanderfolgende Verabreichung der Menge von therapeutischem Mittel (a) und der Menge von therapeutischem Mittel (b) umfasst.

11. Pharmazeutische Kombination, umfassend (S)-Pyrrolidin-1,2-dicarbonsäure-2-amid-1-({4-methyl-5-[2-(2,2,2-trifluor-1,1-dimethylethyl)pyridin-4-yl]thiazol-2-yl}amid) (VERBINDUNG D) oder ein pharmazeutisch unbedenkliches Salz davon und einen c-Met-Rezeptortyrosinkinase-Inhibitor 2-Fluor-N-methyl-4-[7-chinolin-6-yl-methyl)-imidazo[1,2-b]-[1,2,4]triazin-2-yl]benzamid oder ein pharmazeutisch unbedenkliches Salz davon.

12. *(S)*-Pyrrolidin-1,2-dicarbonsäure-2-amid-1-({4-methyl-5-[2-(2,2,2-trifluor-1,1-dimethylethyl)-pyridin-4-yl]thiazol-2-yl}amid) (VERBINDUNG D) oder ein pharmazeutisch unbedenkliches Salz davon zur Verwendung bei der Behandlung einer proliferativen Krankheit mit Dysregulation von c-Met und/oder dem HGF/c-Met-Signalweg durch HGF/c-Met-Gen-Überexpression oder -Amplifikation, HGF-abhängiger autokriner und parakriner Aktivierung und/oder c-Met-Rezeptor-Aktivierung durch eine genetische Mutation in Kombination mit mindestens einem c-Met-Rezeptortyrosinkinase-Inhibitor oder einem pharmazeutisch unbedenklichen Salz davon.

13. Verbindung zur Verwendung nach Anspruch 12, wobei es sich bei dem c-Met-Rezeptortyrosinkinase-Inhibitor um 2-Fluor-N-methyl-4-[7-chinolin-6-yl-methyl)imidazo[1,2-b][1,2,4]triazin-2-yl]benzamid oder ein pharmazeutisch unbedenkliches Salz davon handelt.

14. c-Met-Rezeptortyrosinkinase-Inhibitor, bei dem es sich um 2-Fluor-N-methyl-4-[7-chinolin-6-yl-methyl)imidazo[1,2-b][1,2,4]triazin-2-yl]benzamid oder ein pharmazeutisch unbedenkliches Salz davon handelt, zur Verwendung bei der Behandlung einer proliferativen Krankheit mit Dysregulation von c-Met und/oder dem HGF/c-Met-Signalweg durch HGF/c-Met-Gen-Überexpression oder -Amplifikation, HGF-abhängiger autokriner und parakriner Aktivierung und/oder c-Met-Rezeptor-Aktivierung durch eine genetische Mutation in Kombination mit *(S)*-Pyrrolidin-1,2-dicarbonsäure-2-amid-1-({4-methyl-5-[2-(2,2,2-trifluor-1,1-dimethylethyl)pyridin-4-yl]thiazol-2-yl}amid) (VERBINDUNG D) oder einem pharmazeutisch unbedenklichen Salz davon.

## Revendications

1. Association pharmaceutique comprenant (a) le 2-amide et 1-({4-méthyl-5-[2-(2,2,2-trifluoro-1,1-diméthyl-éthyl)-pyridin-4-yl]-thiazol-2-yl}-amide) de l'acide (*S*)-pyrrolidine-1,2-dicarboxylique (COMPOSÉ D), ou sel pharmaceutiquement acceptable de celui-ci, et (b) au moins un inhibiteur de la tyrosine kinase du récepteur c-Met ou un sel pharmaceutiquement acceptable de celui-ci, pour utilisation dans le traitement d'une maladie proliférative ayant une

dérégulation de c-Met et/ou de la voie de signalisation HGF/c-Met par surexpression ou amplification du gène HGF/c-Met, activation autocrine et paracrine HGF-dépendante et/ou activation du récepteur c-Met par une mutation génétique.

**2.** Association pharmaceutique pour une utilisation selon la revendication 1, dans laquelle l'inhibiteur de la tyrosine kinase du récepteur c-Met est le 2-fluoro-N-méthyl-4-[7-quinoléin-6-yl-méthyl)-imidazo[1,2-b][1,2,4]triazin-2-yl]benzamide, ou un sel pharmaceutiquement acceptable de celui-ci.

**3.** Association pharmaceutique pour une utilisation selon la revendication 1 ou la revendication 2, la maladie proliférative étant un cancer.

**4.** Association pharmaceutique pour une utilisation selon l'une quelconque des revendications 1 à 3, la maladie proliférative étant un cancer choisi dans le groupe consistant en les tumeurs bénignes et malignes du sein, de la vessie, du col de l'utérus, un cholangiocarcinome, les tumerurs colorectales, de l'œsophage, gastriques, de la tête et du cou, du rein, du foie, du poumon, nasopharyngiennes, de l'ovaire, du pancréas, de la prostate, de la thyroïde, endométriales, les sarcomes du système musculosquelettique, les sarcomes des sarcomes des tissus mous, le myélome multiple, les lymphomes, la leucémie à lymphocytes T matures, la leucémie myélogène aiguë, la leucémie myéloïde chronique, les glioblastomes, les astrocytomes, un mélanome, un mésothélioma et la tumeur de Wilm.

**5.** Association pharmaceutique pour une utilisation selon l'une quelconque des revendications 1 à 4, la maladie proliférative étant un cancer du poumon (par exemple, un cancer du poumon non à petites cellules) ou un glioblastome.

**6.** Association pharmaceutique pour une utilisation selon l'une quelconque des revendications 1 à 5, l'administration de l'association étant simultanée, séparée ou séquentielle.

**7.** Composition pharmaceutique comprenant une association pharmaceutique selon la revendication 1 ou la revendication 2 et au moins un véhicule pharmaceutiquement acceptable.

**8.** Association pharmaceutique pour une utilisation selon la revendication 1 dans un procédé d'inhibition de la formation de métastases chez un sujet ayant un cancer, qui comprend l'administration au sujet en ayant besoin d'une quantité pharmaceutiquement efficace d'une association pharmaceutique selon la revendication 1.

**9.** Association pharmaceutique pour une utilisation dans un procédé selon la revendication 8, la maladie proliférative étant un cancer choisi dans le groupe consistant en les tumeurs bénignes et malignes du sein, de la vessie, du col de l'utérus, un cholangiocarcinome, les tumeurs colorectales, de l'œsophage, gastriques, de la tête et du cou, du rein, du foie, du poumon, nasopharyngiennes, de l'ovaire, du pancréas, de la prostate, de la thyroïde, endométriales, les sarcomes du système musculosquelettique, les sarcomes des sarcomes des tissus mous, le myélome multiple, les lymphomes, la leucémie à lymphocytes T matures, la leucémie myélogène aiguë, la leucémie myéloïde chronique, les glioblastomes, les astrocytomes, un mélanome, un mésothélioma et la tumeur de Wilm.

**10.** Association pharmaceutique pour une utilisation dans un procédé selon la revendication 9, le traitement comprenant l'administration de la quantité de l'agent thérapeutique (a) et de la quantité de l'agent thérapeutique (b) séparément ou séquentiellement.

**11.** Association pharmaceutique comprenant le 2-amide et 1-({4-méthyl-5-[2-(2,2,2-trifluoro-1,1-diméthyl-éthyl)-pyridin-4-yl]-thiazol-2-yl}-amide) de l'acide (*S*)-pyrrolidine-1,2-dicarboxylique (COMPOSÉ D), ou un sel pharmaceutiquement acceptable de celui-ci, et un inhibiteur de la tyrosine kinase du récepteur c-Met 2-fluoro-N-méthyl-4-[7-quinoléin-6-yl-méthyl)-imidazo[1,2-b][1,2,4]triazin-2-yl]benzamide, ou un sel pharmaceutiquement acceptable de celui-ci.

**12.** 2-amide et 1-({4-méthyl-5-[2-(2,2,2-trifluoro-1,1-diméthyl-éthyl)-pyridin-4-yl]-thiazol-2-yl}-amide) de l'acide (*S*)-pyrrol idine-1,2-dicarboxylique (COMPOSÉ D), ou sel pharmaceutiquement acceptable de celui-ci, pour une utilisation dans le traitement d'une maladie proliférative ayant une dérégulation de c-Met et/ou de la voie de signalisation HGF/c-Met par surexpression ou amplification du gène HGF/c-Met, activation autocrine et paracrine HGF-dépendante et/ou activation du récepteur c-Met par une mutation génétique, en combinaison avec au moins un inhibiteur de la tyrosine kinase du récepteur c-Met ou un sel pharmaceutiquement acceptable de celui-ci.

**13.** Composé pour une utilisation selon la revendication 12, l'inhibiteur de la tyrosine kinase du récepteur c-Met étant le 2-fluoro-N-méthyl-4-[7-quinoléin-6-yl-méthyl)-imidazo[1,2-b][1,2,4]triazin-2-yl]benzamide, ou un sel pharmaceutiquement acceptable de celui-ci.

**14.** Inhibiteur de la tyrosine kinase du récepteur c-Met qui est le 2-fluoro-N-méthyl-4-[7-quinoléin-6-yl-méthyl)-imidazo[1,2-b][1,2,4]triazin-2-yl]benzamide, ou sel pharmaceutiquement acceptable de celui-ci, pour une utilisation dans le traitement d'une maladie proliférative ayant une dérégulation de c-Met et/ou de la voie de signalisation HGF/c-Met par surexpression ou amplification du gène HGF/c-Met, activation autocrine et paracrine HGF-dépendante, et/ou activation du récepteur c-Met par une mutation génétique, en combinaison avec le 2-amide et 1-({4-méthyl-5-[2-(2,2,2-trifluoro-1,1-diméthyl-éthyl)-pyridin-4-yl]-thiazol-2-yl}-amide) de l'acide (*S*)-pyrrolidine-1,2-dicarboxylique (COMPOSÉ D), ou un sel pharmaceutiquement acceptable de celui-ci.

FIGURE 1

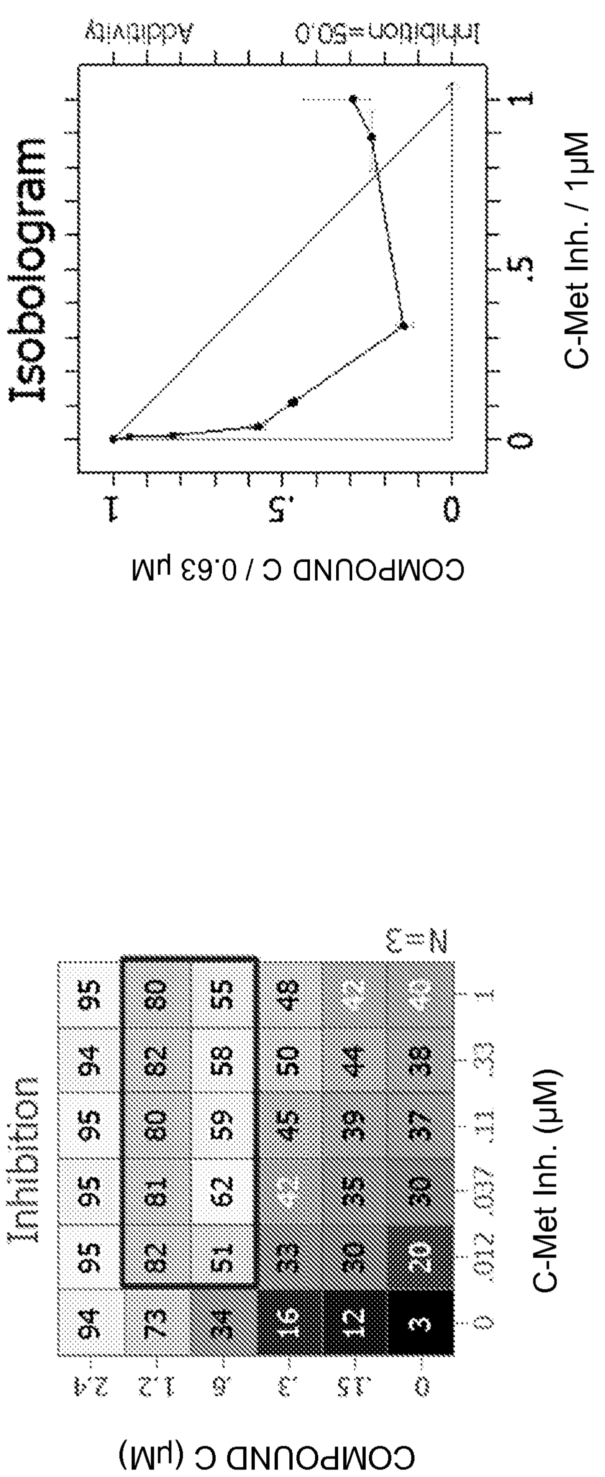
Isobologram
COMPOUND C / 0.63 µM
C-Met Inh. / 1µM
Additivity
Inhibition=50.0
Inhibition
COMPOUND C (µM)
C-Met Inh. (µM)
N=3
2.4 1.2 .6 .3 .15 0
0 .012 .037 .11 .33 1
94 95 95 95 94 95
73 82 81 80 82 80
34 51 62 59 58 55
16 33 42 45 50 48
12 30 35 39 44 42
3 20 30 37 38 40

FIGURE 2

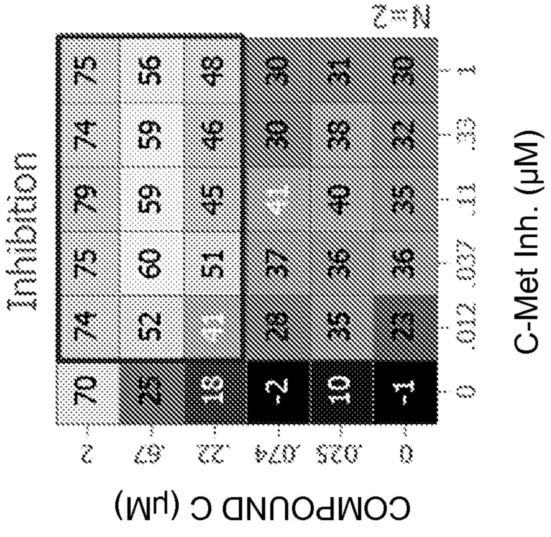
Inhibition
COMPOUND C (µM)
2
.67
.22
.074
.025
0
C-Met Inh. (µM)
0
.012
.037
.11
.33
1
N=2
70 74 75 79 74 75
25 52 60 59 59 56
18 41 51 45 46 48
-2 28 37 41 30 30
10 35 36 40 38 31
-1 23 36 35 32 30

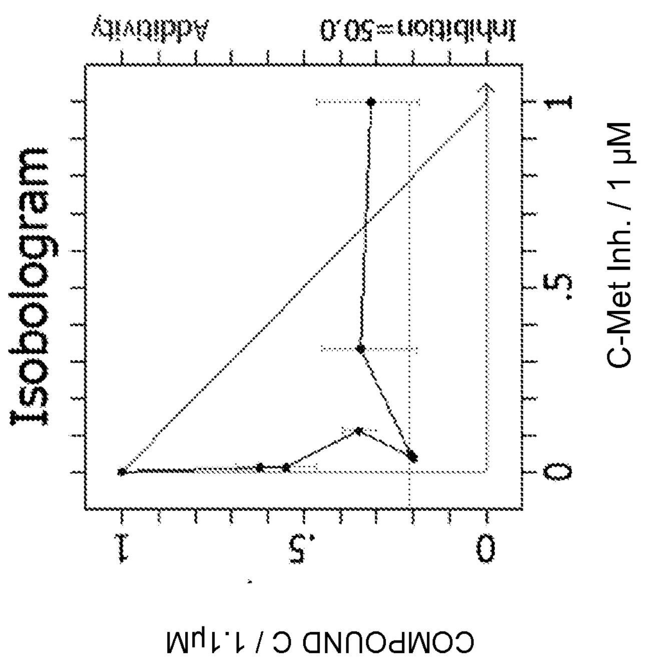
Isobologram
COMPOUND C / 1.1µM
C-Met Inh. / 1 µM
Additivity
Inhibition=50.0
1
.5
0

FIGURE 3

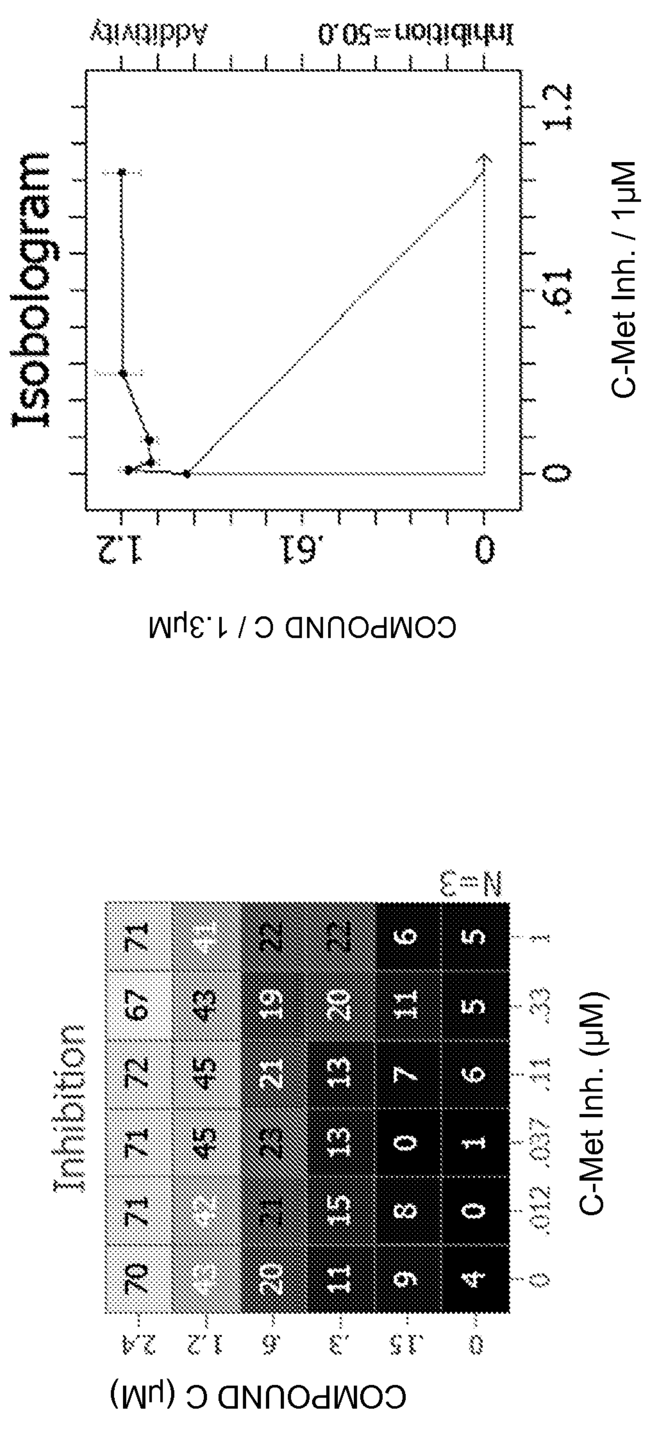
Inhibition
COMPOUND C (μM)
2.4 1.2 .6 .3 .15 0
70 71 71 72 67 71
45 42 45 45 43 41
20 21 23 21 19 22
11 15 13 13 20 22
9 8 0 7 11 6
4 0 1 6 5 5
0 .012 .037 .11 .33 1
C-Met Inh. (μM)
N=3
Isobologram
Additivity
Inhibition=50.0
COMPOUND C / 1.3μM
1.2 .61 0
0 .61 1.2
C-Met Inh. / 1μM

FIGURE 4

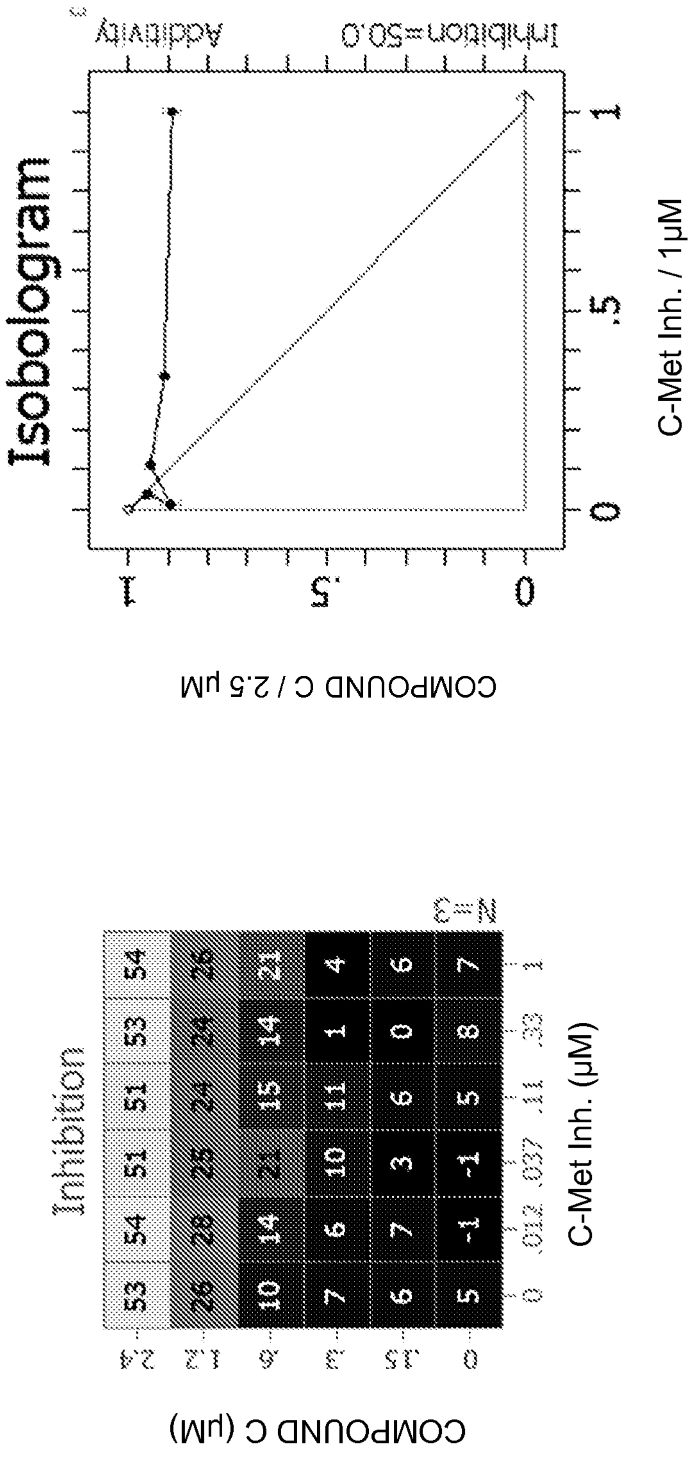
Isobologram
Additivity
Inhibition=50.0
COMPOUND C / 2.5 µM
C-Met Inh. / 1µM
1
.5
0
Inhibition
N=3
COMPOUND C (µM)
2.4
1.2
.6
.3
.15
0
C-Met Inh. (µM)
0
.012
.037
.11
.33
1
53 54 51 51 53 54
26 28 25 24 24 26
10 14 21 15 14 21
7 6 10 11 1 4
6 7 3 6 0 6
5 -1 -1 5 8 7

FIGURE 5

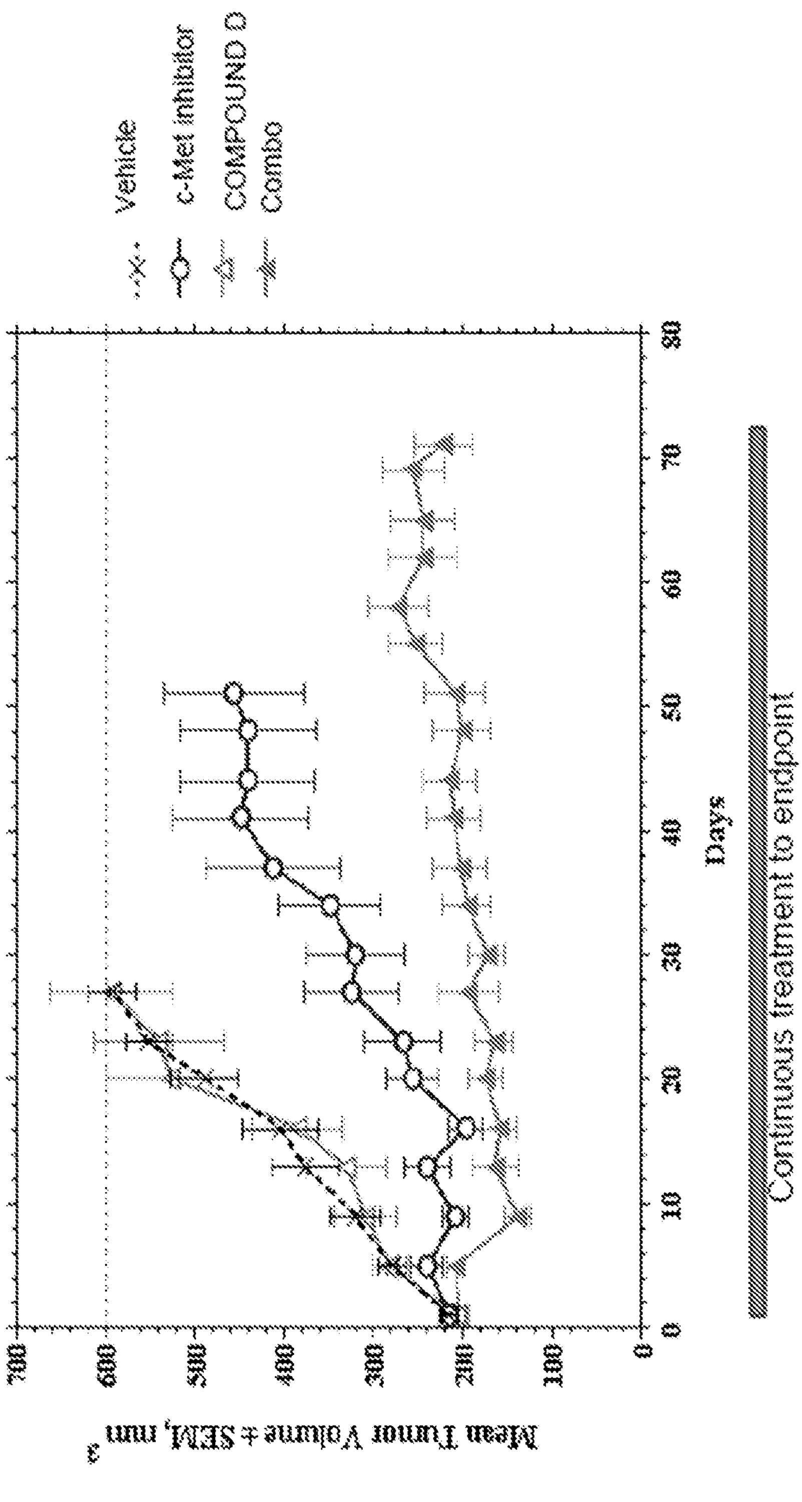
Mean Tumor Volume ± SEM, mm³
700
600
500
400
300
200
100
0
0
10
20
30
40
50
60
70
80
Days
Vehicle
c-Met inhibitor
COMPOUND D
Combo
Continuous treatment to endpoint

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012062694 A
- US 2010267742 A
- WO 2010029082 A
- WO 2008064157 A
- WO 2009143211 A

### Non-patent literature cited in the description


- **BIRCHMEIER, C. et al.** *Nat. Rev. Mol. Cell Biol.,* 2003, vol. 4 (12), 915-925
- **CHRISTENSEN, J. G. et al.** *Cancer Lett.,* 2005, vol. 225 (1), 1-26
- **CHRISTENSEN, J.G. et al.** *Cancer Lett.,* 2005, vol. 225 (1), 1-26
- **CORSO, S. et al.** *Trends in Mol. Med.,* 2005, vol. 11 (6), 284-292
- **BIRCHMEIER, C. et al.** *Nat. Rev. Mol. Cell. Biol.,* 2003, vol. 4 (12), 915-925
- **BOCCACCIO, C. et al.** *Nat. Rev. Cancer,* 2006, vol. 6 (8), 637-645
- **VANHAESEBROECK et al.** *Annu. Rev. Biochem,* 2001, vol. 70, 535
- **KATSO et al.** *Annu. Rev. Cell Dev. Biol.,* 2001, vol. 17, 615
- **FRUMAN et al.** *Annu Rev. Biochem.,* 1998, vol. 67, 481
- **SUIRE et al.** *Curr. Biol.,* 2005, vol. 15, 566
- **STEPHENS et al.** *Cell,* 1997, vol. 89, 105
- **KATSO et al.** *Annu. Rev. Cell Dev. Biol.,* 2001, vol. 17, 615-675
- **CANTLEY et al.** *Cell,* 1991, vol. 64, 281
- **ESCOBEDO ; WILLIAMS.** *Nature,* 1988, vol. 335, 85
- **FANTL et al.** *Cell,* 1992, vol. 69, 413
- **BARTHOLOMEUSZ et al.** *reported in Expert Opin. Ther. Targets,* 2012, vol. 16 (1), 121
- **GONG et al.** *International journal of proteomics 2011,* 2011, vol. 1
- **HOLFORD, N. H. G. ; SCHEINER, L. B.** *Clin. Pharmacokinet.,* 1981, vol. 6, 429-453
- **LOEWE, S. ; MUISCHNEK, H.** *Arch. Exp. Pathol Pharmacol.,* 1926, vol. 114, 313-326
- **CHOU, T. C. ; TALALAY, P.** *Adv. Enzyme Regul.,* 1984, vol. 22, 27-55
- The Handbook of Pharmaceutical Excipients. American Pharmaceuticals Association, 2003
- Remington: the Science and Practice of Pharmacy. Lippincott Williams & Wilkins, 2003
- **LOEWE S.** *Ergebn Physiol,* 1928, vol. 27, 47-187
- **BERENBAUM MC.** *J Theor Biol,* 1985, vol. 114, 413-431
- **LEHAR J et al.** Synergistic drug combinations tend to improve therapeutically relevant selectivity. *Nature Biotechnology,* 2009, vol. 27, 659-66